# EUROPEAN PATENT APPLICATION

(11) **EP 2 239 261 A1**
(43) Date of publication of application: **13.10.2010**
(21) Application number: 09707958.6
(22) Date of filing: 06.02.2009
(51) Int. Cl.: C07D 487/04, A61K 31/5377, A61K 31/541, A61P 35/00, A61P 43/00

(54) **PYRROLOPYRIMIDIN DERIVATIVE FOR USE AS PI3K INHIBITOR, AND USE THEREOF**

(30) Priority: 07.02.2008 JP 2008027423
(71) Applicant: Chugai Seiyaku Kabushiki Kaisha, Kita-ku Tokyo 115-8543 (JP)
(72) Inventor: EBIIKE, Hirosato, Kanagawa 247-8530 (JP); OHWADA, Jun, Kanagawa 247-8530 (JP); KOYAMA, Kohei, Kanagawa 247-8530 (JP); MURATA, Takeshi, Kanagawa 247-8530 (JP); HONG, Woo Sang, Gyeonggi-do, 445-380 (KR)
(74) Representative: HOFFMANN EITLE
(86) International application number: PCT/JP2009/052017
(87) International publication number: WO 2009/099163

(57) **Abstract**

A preventive or therapeutic agent of a proliferative disease such as cancer, having superior PI3K inhibitory effects, superior cell proliferation inhibitory action as well as superior stability in a body and water solubility, is provided.

A compound represented by formula (I): 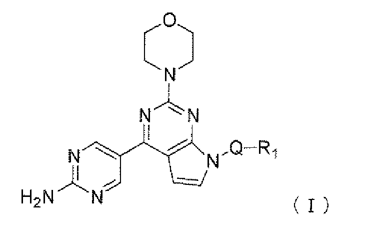 [wherein, Q represents a linking group represented by -X-Y-; X represents a single bond -CO-, -CONH-, -CON(C₁₋₄ alkyl)-, -CS-, -CSNH-, -CSN(C₁₋₄ alkyl)-, or -SO₂-; Y represents a single bond, arylene or heteroarylene; provided that X and Y are not simultaneously single bonds; and R₁ represents -C₀₋₆ alkylene-(A)ₘ-C₁₋₆ alkyl, or C₀₋₆ alkylene-(A)ₘ-C₀₋₆ alkylene-(heterocycle)]
or a pharmaceutically acceptable salt thereof.

## Description

### TECHNICAL FIELD

The present invention relates to a novel condensed pyrimidine derivative and a pharmaceutically acceptable salt thereof, a pharmaceutical composition containing the same, and synthetic intermediates and the like thereof.

### BACKGROUND ART

Phosphatidylinositol 3-kinase (PI3K) is known as a kind of phosphorylases of phosphatidylinositol that phosphorylates 3-position of an inositol ring, and is expressed over a wide range throughout the body. The PI3K is known to be activated by stimulation including growth factors, hormones and the like, activate Akt and PDK1, and be involved in survival signals that inhibit cell death,cytoskeleton, glucose metabolism, vesicular transport and the like. In addition, the phosphatidylinositols phosphorylated at position 3 that are formed by PI3K function as messengers of these signal transduction system (Phosphatidylinositol 3-kinases in tumor progression. Eur. J. Biochem. 268, 487-498 (2001); Phosphoinositide 3-kinase: the key switch mechanism in insulin signaling. Biochem. J. 333, 471-490 (1998); Distinct roles of class I and class III phosphatidylinositol 3-kinase in phagosome formation and maturation. J. C. B., 155(1), 19-25 (2001) and the like).

PI3K is categorized into three classes consisting of Class I, Class II and Class III according to the type of phosphatidylinositols serving as a substrate.

Although Class I enzymes form phosphatidylinositol (3,4,5)-triphosphate [PI(3,4,5)P3] by using phosphatidylinositol (4,5)-bisphosphate [PI(4,5)P2] as a substrate in vivo, it is able to use phosphatidylinositol (PI) and phosphatidylinositol (4)-phosphate [PI(4)P] as a substrates in vitro. Further, Class I enzymes are categorized into Class Ia and Ib according to the activation mechanism. Class Ia includes the p110α, p110β and p110δ subtypes, and each forms a heterodimer complex with a regulatory subunit (p85) and is activated by a tyrosine kinase receptor and the like. Class Ib includes a p110γ subtype that is activated by the βγ subunit (Gβγ) of a trimer G protein, and forms a heterodimer with a regulatory subunit (p101).

Class II enzymes include the PI3KC2α, C2β and C2γ subtypes, that use PI and PI(4)P as substrates. These enzymes have a C2 domain on the C terminal, and regulatory subunits as observed for Class I enzymes have not yet to be discovered.

Class III enzymes only use PI as a substrate, and are reported to be involved in membrane transport control as a result of interaction between p 150 and human Vps34, a human homolog of Vps34 isolated from yeast.

As a result of analyses using these PI3K knockout mice, p110δ in Class Ia has been reported to be involved in the differentiation and function of T cells and B cells, while p110γ in Class Ib has been reported to be involved in abnormalities of migration of neutrophils, mast cells, platelets and myocardial cells (Phosphoinositide 3-kinase signaling - which way to target? Trends in Pharmacological Science, 24(7), 366-376 (2003)).

On the basis of these results, the targeting of p110δ and p110γ of Class I is expected to be useful against autoimmune diseases, inflammations, asthma, heart disease and the like.

Recently, a gene amplification of PIK3CA encoding p110α, constitutive activation due to mutation, and high expression of p 110α at the protein level have been reported in numerous types of cancers (and particularly ovarian cancer, colon cancer and breast cancer). As a result, inhibition of apoptosis by constitutive activation of survival signals is believed to be partially responsible for the mechanism of tumorigenesis (PIK3CA is implicated as an oncogene in ovarian cancer. Nature Genet. 21, 99-102, (1999); High frequency of mutations of the PIK3CA gene in human cancers. Science, 304, 554, (2004); Increased levels of phosphoinositol 3-Kinase activity in colorectal tumors. Cancer, 83, 41-47 (1998)).

In addition, the deletion or mutation of PTEN, a phospholipid phosphatase which utilizes PI(3,4,5)P3 as a substrate that is one of the products of PI3K, has been reported in numerous cancers. Since PTEN functions as a suppressor of PI3K as a result of using PI(3,4,5)P3 as a substrate, deletion or mutation of PTEN is thought to lead to activation of PI3K in the PI3K signal.

On the basis of these reasons, useful anticancer action is expected to be obtained by inhibiting the activity of p110α in particular in cancers with elevated PI3K activity.

In this manner, Wortmannin (Non-Patent Document 1) and LY294002 (Non-Patent Document 2) are known to be specific inhibitors of PI3K, that are expected to be useful in the fields of immune diseases, anti-inflammatory agents, anticancer agents and the like.

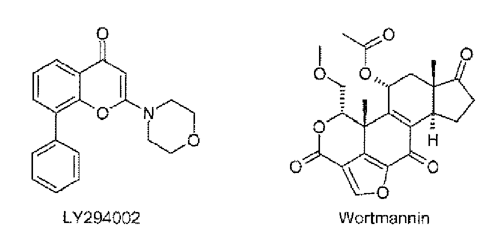

Although numerous compounds having PI3K inhibitory action have recently been reported, none have yet to be commercially available as pharmaceuticals based on the PI3K inhibitory action thereof, thus creating the desire for the prompt development of anticancer agents and the like having PI3K inhibitory action that are able to be used clinically.

In contrast, with regard to 5-(2-morpholin-4-yl-7H-pyrrolo[2,3-d]pyimidin-4-yl)-pyrimidin-2-yl amine derivative which are represented by the compound of the present invention of the general formula (I) to be described later, the mother structure *per se* has heretofore not been known, and the usefulness of these derivative as anticancer agents and the like having PI3K inhibitory action is also not known.
Patent Document 1: US Patent No. 5378700
Non-Patent Document 1: H. Yano et al., J. Biol. Chem., 268, 25846, 1993
Non-Patent Document 2: C.J. Vlahos et al., J. Biol. Chem., 269, 5241, 1994

### DISCLOSURE OF THE INVENTION

### Problems to be Solved by the Invention

As a result of conducting extensive studies to develop a compound that is useful as an anticancer agent having inhibitory activity on PI3K and superior safety, the inventors of the present invention found that a 5-(2-morpholin-4-yl-7H-pyrrolo[2,3-d]pyrimidin-4-yl)-pyrimidin-2-ylamine derivative having the encircled portion of general formula (I') indicated below as a mother structure, has a superior PI3K inhibitory effect and a cell proliferation inhibitory action, as well as superior stability in a body and water solubility, allowing it to be a useful drug for the prevention or treatment of cancer, thereby leading to completion of the present invention. Further, the inventors also found compounds useful as a synthesis intermediate thereof, leading to completion of the present invention.

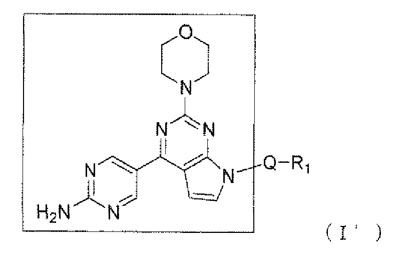

### Means for Solving the Problems

Namely, the present invention provides a compound represented by formula (I) indicated below or a pharmaceutically acceptable salt thereof, a preparation process of the compound represented by formula (I) and a synthesis intermediate thereof, and a pharmaceutical composition comprising the compound of formula (I) or a pharmaceutically acceptable salt thereof.

The present invention relates to a compound represented by formula (I): 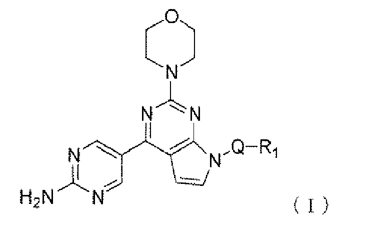 [wherein,
Q represents a linking group represented by -X-Y-;
X represents a single bond, -CO-, -CONH-, -CON(C₁₋₄ alkyl)-, -CS-, - CSNH-, -CSN(C₁₋₄ alkyl)-, or -SO₂-;
Y represents a single bond, arylene or heteroarylene (the arylene and heteroarylene may be unsubstituted or substituted at 1 to 4 locations by -halogen, -C₁₋₆ alkyl, -OH, or -OC₁₋₆ alkyl);
provided that X and Y are not simultaneously single bonds;
R₁ represents -C₀₋₆ alkylene-(A)ₘ-C₁₋₆ alkyl, or C₀₋₆ alkylene-(A)ₘ-C₀₋₆ alkylene-(heterocycle);
A represents -CO-, -CS-, -CONH-, -CON(C₁₋₄ alkyl)-, -CSNH-, -CSN(C₁₋₄ alkyl)-, -NH-, or N(C₁₋₄ alkyl)-;
m represents 0 or 1;
the aforementioned -(heterocycle) is heteroaryl, or a group represented by formula (a); 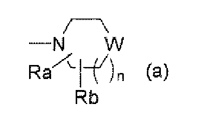 wherein Rₐ and R_{b} are the same or different and represent a hydrogen atom, -C₁₋₆ alkyl, -halogen, -OH, or -OC₁₋₆ alkyl;
W represents -CR_{c}R_{d}-, -O-, -S-, -SO-, -SO₂-, or -NRₑ-;
n represents 0 or 1;
R_{c} and R_{d} are the same or different and represent a hydrogen atom, - halogen, -C₁₋₆ alkyl, -OH, -OC₁₋₆ alkyl, or heteroaryl;
Rₑ represents a hydrogen atom, -C₁₋₆ alkyl, -OH, -OC₁₋₆ alkyl, or heteroaryl (-C₁₋₆ alkyl and -OC₁₋₆ alkyl in R_{c}, R_{d} and Rₑ may be substituted by -halogen or -OH)], or a pharmaceutically acceptable salt thereof

The present invention also relates to a process for preparing a compound represented by the aforementioned formula (I): 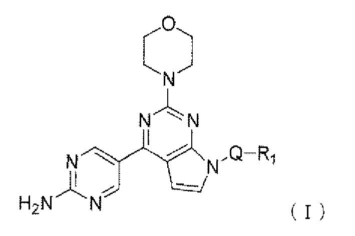 [wherein, Q and R₁ are the same as defined above]
which comprises the step of reacting the compound represented by formula (VIa): 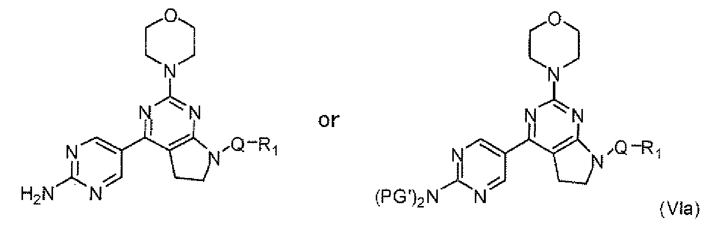 [wherein, Q and R₁ are the same as defined above; and PG' represents an amino group-protecting group]
with an oxidizing agent and may further comprise the step of removing the amino group-protecting group.

The present invention further relates to a process for preparing a compound represented by formula (VIa): 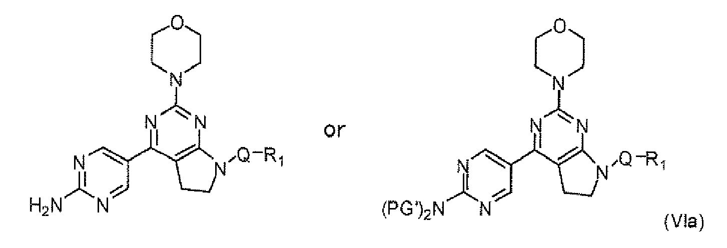 [wherein, Q and R₁ are the same as defined above; and PG' represents an amino group-protecting group],
the preparation process comprising the steps of reacting a compound represented by formula (Va): 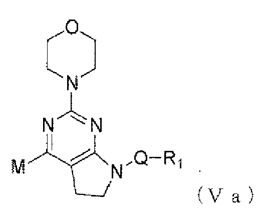 [wherein, M represents a leaving group; and Q and R₁ are the same as defined above] with a boronic acid derivative represented by the following formula: 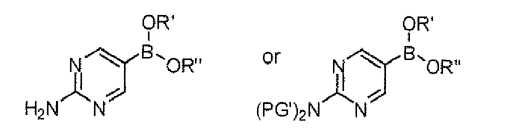 [wherein, R' and R" each independently represent a hydrogen atom or C₁₋₆ alkyl, or R' and R" may together form -C₂₋₃ alkylene-, where -C₂₋₃ alkylene- may be substituted at 1 to 4 locations by -methyl; and PG' represents an amino group-protecting group] in the presence of a palladium catalyst and a ligand, to obtain a compound represented by formula (VIa).
The present invention further relates to a pharmaceutical composition comprising as an active ingredient the compound represented by the aforementioned formula (I) or a pharmaceutically acceptable salt thereof.

### Effects of the Invention

Since the compound of the present invention represented by formula (I) has superior PI3K inhibitory effects, superior cell proliferation inhibitory action and superior stability in a body and water solubility, it can be used as a preventive agent or therapeutic agent for a proliferative disease such as cancer. In addition, some of the compounds among the compounds represented by formula (I) are also useful as synthesis intermediates of other compounds. In addition, the compound represented by formula (VIa) is useful as a synthesis intermediate for obtaining the compound represented by the aforementioned formula (I).

### BEST MODE FOR CARRYING OUT THE INVENTION

The following provides an explanation of the compound of the present invention, a preparation process thereof and a pharmaceutical containing that compound.

The terms used in the present specification are defined as described below.
In the present specification, -C₁₋₆ alkyl refers to a linear or branched, monovalent saturated hydrocarbon group having 1 to 6 carbon atoms, and a preferable example of -C₁₋₆ alkyl is an alkyl group having 1 to 4 carbon atoms (-C₁₋₄ alkyl). Specific examples of -C₁₋₆ alkyl include -methyl, -ethyl, -n-propyl, -isopropyl, -n-butyl, -isobutyl, -t-butyl, -sec-butyl, -n-pentyl, -n-hexyl, -1-methylpentyl, -2-methylpentyl, -3-methylpentyl, -4-methylpentyl, -1,1-dimethylbutyl, -1,2-dimethylbutyl, -1,3-dimethylbutyl, -2,2-dimethylbutyl, -2,3-dimethylbutyl, -3,3-dimethylbutyl, -1-ethylbutyl, -2-ethylbutyl, -1,1,2-trimethylpropyl and -1,2,2-trimethylpropyl, more preferable examples include -methyl, -ethyl, n-propyl and isopropyl, and particularly preferable examples include -methyl, and -ethyl.
In the present specification, -C₀₋₆ alkylene- refers to a single bond (the number of carbon atom is 0) or a linear, divalent saturated hydrocarbon group having 1 to 6 carbon atoms (-C₁₋₆ alkylene-). Specific examples of -C₁₋₆ alkylene- include methylene, ethylene, propylene, butylene, pentylene and hexylene. Preferably, an alkylene group having 0 to 4 carbon atoms (-C₀₋₄ alkylene-) may be mentioned and specific examples thereof include a single bond, methylene, ethylene, propylene and butylene. In addition, the -C₁₋₆ alkylene- may be substituted by a group selected from -C₁₋₆ alkyl, -OH, -CONH₂, -NH₂, -NH(C₁₋₆ alkyl) and -N(C₁₋₆ alkyl)₂. The -C₀₋₆ alkylene- is preferably a single bond or -C₁₋₆ alkylene which may be substituted by a group selected from -OH, -methyl and -dimethylamino, or more preferably a single bond or -C₁₋₄ alkylene- which is unsubstituted.

In the present specification, -halogen refers to a monovalent group derived from a halogen atom (for example, F, Cl, Br or I). Examples include -F, -Cl, -Br and -I, preferably -F and -Cl, and more preferably -F.

In the present specification, arylene refers to a divalent cyclic group comprising a mono- or bicyclic aromatic hydrocarbon ring. The number of atoms that compose the ring is, for example, 5 to 10, and preferably the number of carbon atoms are 6 to 10. As specific examples of arylene, phenylene and naphtylene may be mentioned, more preferably, phenylene, even more preferably, when Y represents arylene, the two linkers of the linker from Y to X and the linker from Y to R₁ are in the relation of the meta position, or the para position.
The arylene may be unsubstituted or substituted, for example, at 1 to 4 locations by -halogen, -C₁₋₆ alkyl, -OH, or -OC₁₋₆ alkyl.

In the present specification, heteroarylene refers to a divalent cyclic group derived from a mono- or bicyclic aromatic heterocycle comprising a heteroatom. In addition to ring members in the form of carbon atoms, it also contain at least one nitrogen atom, and may additionally contain 1 to 2 heteroatoms selected from nitrogen, oxygen and sulfur. The number of atoms that compose the ring may be preferably 3 to 12, more preferably 5 to 6. Although the ring may be monocyclic or bicyclic, it is preferably monocyclic. Specific examples of heterocycles which derives such heteroarylene include aromatic heterocyclic rings such as pyrrol, pyrazole, imidazole, triazole, oxazole, isoxazole, indazole, thiazole, pyridine, piridazine, pyrimidine, pyrazine, oxazine, triazine, indole, benzimidazole, benzoxazole, benzothiazole, benzopyrazole, quinoline, isoquinoline, quinoxaline, quinazoline, phthalazine, purine or pteridine, more preferably 5- to 6- member aromatic heterocyclic rings such as pyrrol, pyrazole, imidazole, triazole, oxazole, isoxazole, indazole, thiazole, pyridine, piridazine, pyrimidine, pyrazine, oxazine, or triazine. Even more preferable examples include pyridine, piridazine, pyrimidine, and pyrazine, and a particularly preferable example is pyridine.
When heteroarylene is derived from a 5-member aromatic heterocyclic ring, the substitution positions of the two linkers are preferably position 1 and position 3, position 2 and position 4, or position 3 and position 5, when heteroarylene is derived from a 6-member aromatic heterocyclic ring, and when Y represents heteroarylene, the substitution positions of the two linkers extending from Y to X and from Y to R₁ are preferably in the relations of position 2 and position 4, position 2 and position 5, position 2 and position 6, or position 3 and position 5.
The heteroarylene may be unsubstituted or substituted, for example, at 1 to 4 locations by -halogen, -C₁₋₆ alkyl, -OH, or -OC₁₋₆ alkyl.
In the present specification, heteroaryl refers to a monovalent cyclic group derived from a mono- or bicyclic aromatic heterocycle comprising a heteroatom. In addition to ring members in the form of carbon atoms, it also contain at least one nitrogen atom, and may additionally contain 1 to 2 heteroatoms selected from nitrogen, oxygen and sulfur. The number of atoms that compose the ring may be preferably 3 to 12, more preferably 5 to 6. Although the ring may be monocyclic or bicyclic, it is preferably monocyclic. Specific examples of heterocycles which derives such heteroaryl include aromatic heterocyclic rings such as pyrrol, pyrazole, imidazole, triazole, oxazole, isoxazole, indazole, thiazole, pyridine, piridazine, pyrimidine, pyrazine, oxazine, triazine, indole, benzimidazole, benzoxazole, benzothiazole, benzopyrazole, quinoline, isoquinoline, quinoxaline, quinazoline, phthalazine, purine or pteridine, more preferably 5- to 6-member aromatic heterocyclic rings such as pyrrol, pyrazole, imidazole, triazole, oxazole, isoxazole, indazole, thiazole, pyridine, piridazine, pyrimidine, pyrazine, oxazine, or triazine. Even more preferable examples include pyridine, piridazine, pyrimidine, and pyrazine, and a particularly preferable example is pyridine.

In the present specification, -(heterocycle) refers to the heteroaryl defined above, or the group represented by the following formula (a): 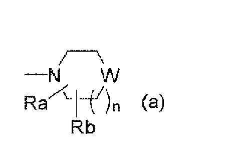 In the formula, Rₐ and R_{b} are the same or different and represent a hydrogen atom, -C₁₋₆ alkyl, -halogen, -OH, or -OC₁₋₆ alkyl;
W represents -CR_{c}R_{d}-, -O-, -S-, -SO-, -SO₂-, or NRₑ-;
n represents 0 or 1;
R_{c} and R_{d} are the same or different and represent a hydrogen atom, - halogen, -C₁₋₆ alkyl, -OH, -OC₁₋₆ alkyl, or heteroaryl;
Rₑ represents a hydrogen atom, -C₁₋₆ alkyl, -OH, -OC₁₋₆ alkyl, or heteroaryl.
The -C₁₋₆ alkyl and -OC₁₋₆ alkyl in R_{c}, R_{d} and Rₑ may be substituted by - halogen, or -OH, preferably substituted at 1 to 3 locations by -F, or unsubstituted, and more preferably, unsubstituted.

Examples of preferable modes of the respective substitutents in the group represented by the aforementioned formula (a) include those indicated below.
Rₐ and R_{b} are the same or different and is preferably a hydrogen atom or C₁₋₄ alkyl.
W is preferably -O-, -S-, -SO₂-, or -NRₑ-.
n is preferably 1.
R_{c} and R_{d} are the same or different and are preferably a hydrogen atom, -C₁₋₄ alkyl, or -OH.
Rₑ is preferably a hydrogen atom, -(C₁₋₄ alkyl which may be substituted by a halogen atom), or pyridyl, more preferably, a hydrogen atom, -(C₁₋₄ alkyl which may be substituted at 1 to 3 locations by -F), or pyridyl, even more preferably, a hydrogen atom, or -C₁₋₄ alkyl.
Examples of preferable modes of the group represented by the aforementioned formula (a) include the groups represented by the formula (a) having the following combinations of the group:
Rₐ and R_{b} are the same or different and are preferably a hydrogen atom, or C₁₋₄ alkyl;
W is preferably -O-, -S-, -SO₂-, or -NRₑ-;
n is preferably 1; and
Rₑ is preferably a hydrogen atom or -C₁₋₄ alkyl.

Specific examples of groups represented by formula (a) include those represented by the following formula (a-1), (a-2), (a-3), (a-4), (a-5), (a-6), (a-7), or (a-8). 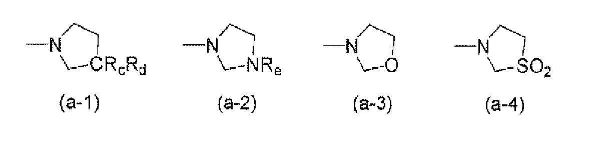 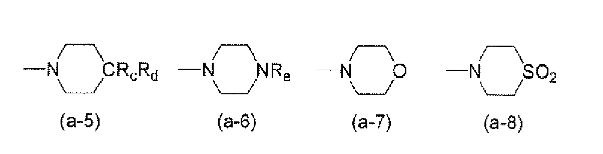 As a group represented by formula (a), more preferably included are those represented by (a-6), (a-7), or (a-8). Here, preferable examples of Rₑ include a hydrogen atom, methyl, ethyl, 2-fluoroethyl, n-propyl, i-propyl, 3,3,3-trifluoro-n-propyl, n-butyl, 4-fluoro-n-butyl, 3-pyridyl, and 4-pyridyl, more preferably, a hydrogen atom, methyl, ethyl, n-propyl, and i-propyl.

The following provides an explanation of the compound represented by formula (I) of the present invention and a pharmaceutically acceptable salt thereof.
The compounds represented by formula (I) of the present invention are as described above. In the formula (I), while Q represents a linking group represented by -X-Y- as described above, preferably, either X or Y is a single bond.

In formula (I), X represents, as described above, a single bond, -CO-, - CONH-, -CON(C₁₋₄ alkyl)-, -CS-, -CSNH-, -CSN(C₁₋₄ alkyl)-, or -SO₂-; and Y represents, as described above, a single bond, arylene or heteroarylene (the arylene and heteroarylene may be unsubstituted or substituted at 1 to 4 locations by -halogen, -C₁₋₆ alkyl, -OH, or -OC₁₋₆ alkyl). X and Y, however, are not simultaneously single bonds.
Preferable modes of X and Y include the following:
X is preferably a single bond, -CO-, -CONH-, -CSNH-, or -SO₂-, more preferably, a single bond, or -SO₂-.
Y is, as described above, a single bond, arylene or heteroarylene (the arylene and heteroarylene may be unsubstituted or substituted at 1 to 4 locations by - halogen, -C₁₋₆ alkyl, -OH, or -OC₁₋₆ alkyl, preferably unsubstituted or substituted by - C₁₋₄ alkyl), more preferably a single bond, phenylene, or pyridinylene (the phenylene and pyridinylene may be unsubstituted or substituted at 1 to 2 locations by methyl or - F), even more preferably a single bond, 1,3-phenylene, 1,4-phenylene, 2-methyl-1,4-phenylene, 2-methyl-1,5-phenylene, 2-fluoro-1,4-phenylene, 3-fluoro-1,4-phenylene, 2,6-difluoro-1,4-phenylene, 2,4-pyridinylene, or 2,5-pyridinylene, particularly preferably a single bond, 1,3-phenylene, 1,4-phenylene, 2-methyl-1,4-phenylene, or 2,4-pyridinylene.

Furthermore, when Y represents arylene or heteroarylene, the arylene or heteroarylene is derived preferably from benzene, pyrrole, pyrazole, imidazole, triazole, oxazole, isoxazole, indazole, thiazole, pyridine, piridazine, pyrimidine, pyrazine, oxazine, triazine, indole, benzimidazole, benzoxazole, benzothiazole, benzopyrazole, quinoline, isoquinoline, quinoxaline, quinazoline, phthalazine, purine, or pteridine, more preferably from benzene or pyridine.

Futhermore, in formula (I), R₁ represents, as described above, -C₀₋₆ alkylene-(A)ₘ-C₁₋₆ alkyl or -C₀₋₆ alkylene-(A)ₘ-C₀₋₆ alkylene-(heterocycle), preferably -C₀₋₄ alkylene-(A)ₘ-C₁₋₄ alkyl or -C₀₋₄ alkylene-(A)ₘ₋C₀₋₄ alkylene-(heterocycle), more preferably -C₁₋₄ alkylene-A-(heterocycle), -A-C₁₋₄ alkylene-(heterocycle), -C₁₋₄ alkylene-A-C₁₋₄ alkylene-(heterocycle), or -C₁₋₄ alkyl, -(heterocycle), even more preferably -C₁₋₄ alkyl, -C₁₋₄ alkylene-(heterocycle), -CO-(heterocycle) or - (heterocycle), particularly preferably, -methyl, -CH₂-(heterocycle), or -(heterocycle). A and m here are the same as defined above, and -(heterocycle) is, as described above, heteroaryl, or is represented by the following formula (a): 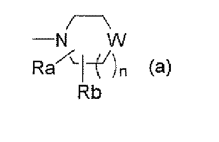 wherein each group in the formula is as defined above.

When R₁ represents -C₀₋₆ alkylene-(A)ₘ-C₀₋₆ alkylene-(heterocycle), heteroaryl represented by the -(heterocycle) is preferably pyridyl; the group of formula (a) represented by -(heterocycle) is preferably a group represented by the following formula (a-1), (a-2), (a-3), (a-4), (a-5), (a-6), (a-7), or (a-8), more preferably, a group represented by the following formula (a-6), (a-7), or (a-8). In the groups represented by the following formulae (a-1) to (a-8), R_{c}, R_{d}, and Rₑ are the same as defined above; R_{c} and R_{d} are preferably a hydrogen atom, -C₁₋₄ alkyl, or -OH, more preferably a hydrogen atom, or -OH; Rₑ is preferably a hydrogen atom, -(C₁₋₄ alkyl which may be substituted by halogen atom), or pyridyl, more preferably, a hydrogen atom, -(C₁₋₄ alkyl which may be substituted at 1 to 3 locations by -F), or pyridyl, even more preferably, a hydrogen atom, or -C₁₋₄ alkyl. Rₑ is particularly preferably, methyl, or ethyl.

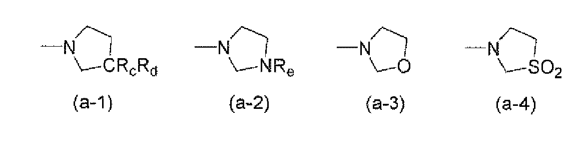

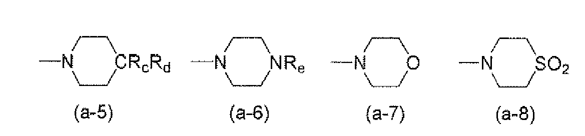

In more preferable modes of A in R₁, A is preferably -CO-, -NH-, -CONH-, or CON(C₁₋₄ alkyl)-, more preferably -CO-, -NH-, -CONH-, or CONMe-.

The compound represented by general formula (I) in more preferable aspect of the present invention is the compound wherein,
X is a single bond, -CO-, -CONH-, -CSNH-, or -SO₂-;
Y is a single bond, phenylene, or pyridinylene; and
R₁ is 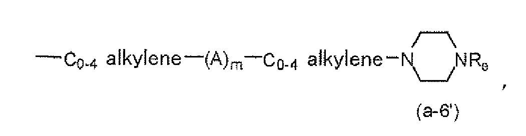 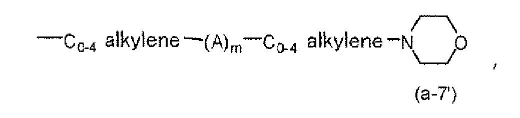 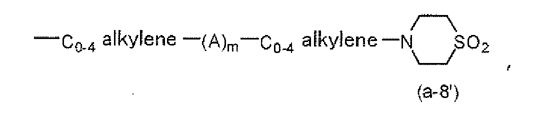 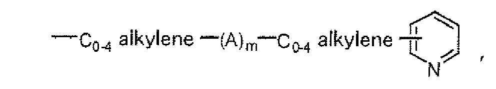 or -C₁₋₄ alkyl
[wherein, A represents -CO-, -NH-, -CONH-, or CONMe-;
m is 0 or 1;
Rₑ represents a hydrogen atom, -(C₁₋₆ alkyl which may be substituted by a halogen atom), or pyridyl].

Specific examples of compounds represented by general formula (I) of the present invention and pharmaceutically acceptable salts thereof include those compounds described below and those compounds described in the following tables (including free forms and salts thereof). However, the present invention should not be limited to these exemplifications.

### Compound of formula (I)

5-{7-[2-(4-ethyl-piperazin-1-yl)-pyridin-4-yl]-2-morpholin -4-yl-7H-pyrrolo[2,3-d]pyrimidin-4-yl}-pyrimidin-2-ylamine;
{3-[4-(2-amino-pyrimidin-5-yl)-2-morpholin-4-yl-pyrrolo [2,3-d]pyrimidin-7-yl]-4-methyl-phenyl}-morpholin-4-yl-methanone;
5-{7-[4-(1,1-dioxo-1λ⁶-thiomorpholin-4-ylmethyl)-phenyl]-2-morpholin-4-yl-7H-pyrrolo[2,3-d]pyrimidin-4-yl}-pyrimidin-2-ylamine; and
5-(7-methanesulfonyl-2-morpholin-4-yl-7H-pyrrolo[2,3-d]pyrimidin-4-yl)-pyrimidin-2-ylamine.

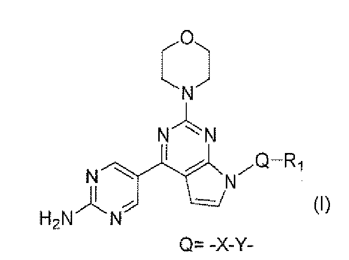

| Example | Compound No. | Structural formula | -X- | -Y- | -R₁ |
|---|---|---|---|---|---|
| 1 | 1-1 | 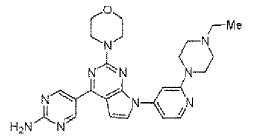 | single bond | 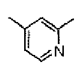 | 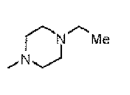 |
| 2 | 2-1 | 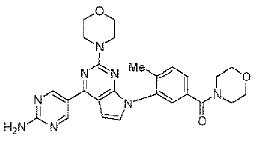 | single bond | 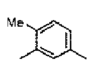 | 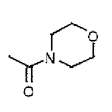 |
| 3 | 3-1 | 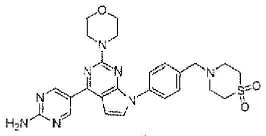 | single bond, | 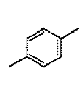 | 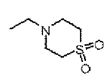 |
| 4 | 4-1 | 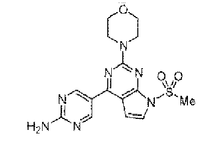 | 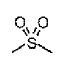 | single bond | -Me |

The following compounds can also be mentioned.

| Compound No. | Structural formula | -X- | -Y- | -R1 |
|---|---|---|---|---|
| 5 | 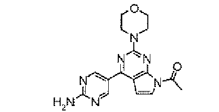 | -CO- | single bond | -Me |
| 6 | 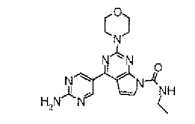 | -CONH- | single bond | -Et |
| 7 | 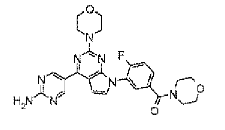 | single bond | 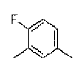 | 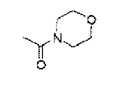 |
| 8 | 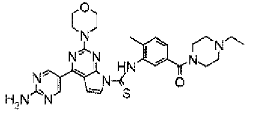 | -CSNH- | 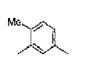 | 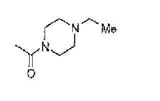 |
| 9 | 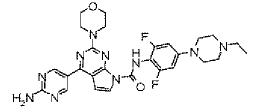 | -CONH- | 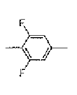 | 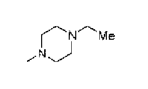 |
| 10 | 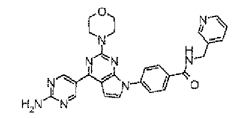 | single bond | 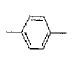 | 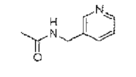 |
| 11 | 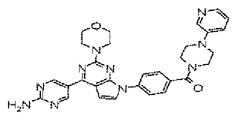 | single bond | 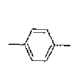 | 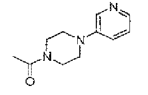 |
| 12 | 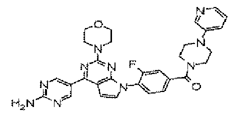 | single bond | 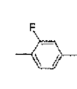 | 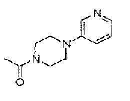 |
| 13 | 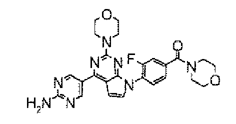 | single bond | 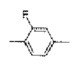 | 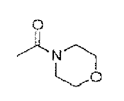 |
| 14 | 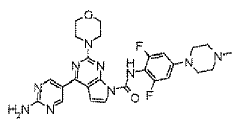 | -CONH- | 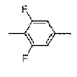 | 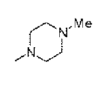 |
| 15 | 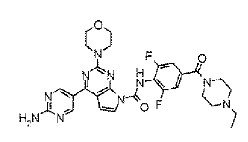 | -CONH- | 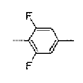 | 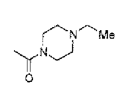 |
| 16 | 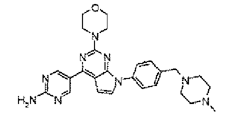 | single bond | 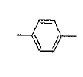 | 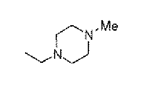 |
| 17 | 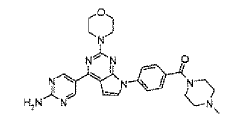 | single bond | 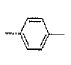 | 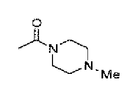 |
| 18 | 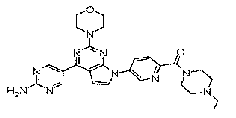 | single bond | 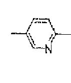 | 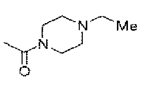 |
| 19 | 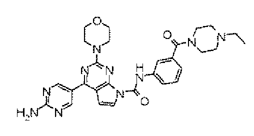 | -CONH- | 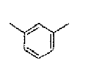 | 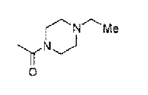 |
| 20 | 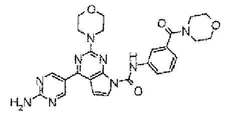 | -CONH- | 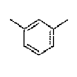 | 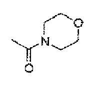 |
| 21 | 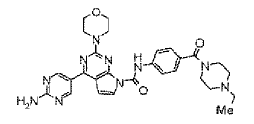 | -CONH- | 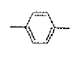 | 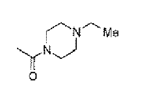 |
| 22 | 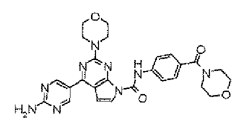 | -CONH- | 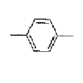 | 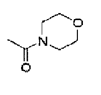 |
| 23 | 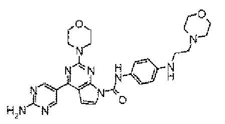 | -CONH- | 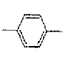 | 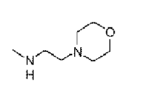 |
| 24 | 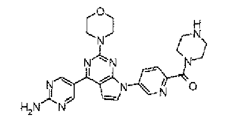 | single bond | 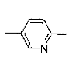 | 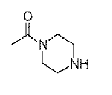 |
| 25 | 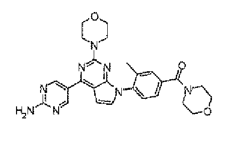 | single bond | 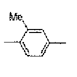 | 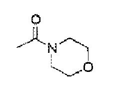 |
| 26 | 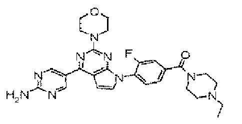 | single bond | 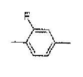 | 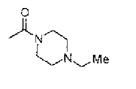 |
| 27 | 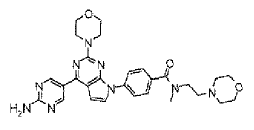 | single bond | 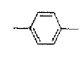 | 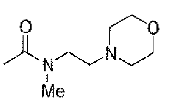 |
| 28 | 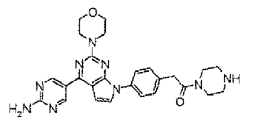 | single bond | 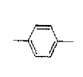 | 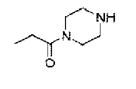 |
| 29 | 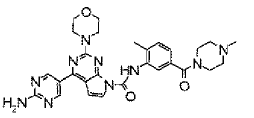 | -CONH- | 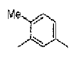 | 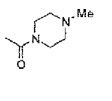 |
| 30 | 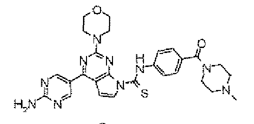 | -CSNH- | 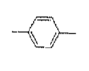 | 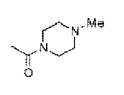 |
| 31 | 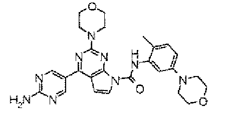 | -CONH- | 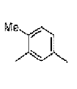 | 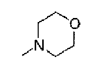 |
| 32 | 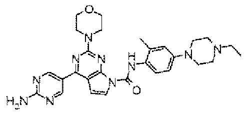 | -CONH- | 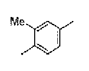 | 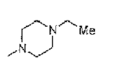 |
| 33 | 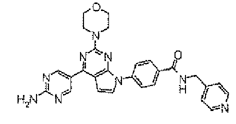 | single bond | 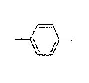 | 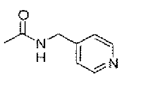 |
| 34 | 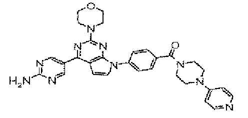 | single bond | 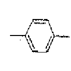 | 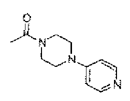 |
| 35 | 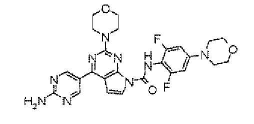 | -CONH- | 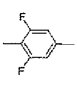 | 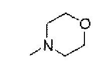 |
| 36 | 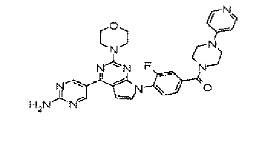 | single bond | 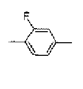 | 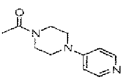 |
| 37 | 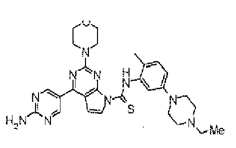 | -CSNH- | 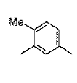 | 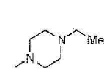 |
| 38 | 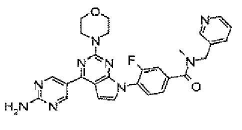 | single bend | 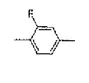 | 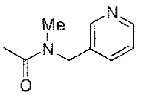 |
| 39 | 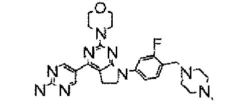 | single bond | 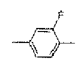 | 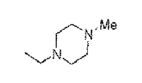 |
| 40 | 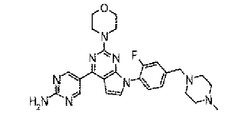 | single bond | 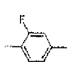 | 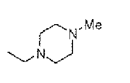 |
| 41 | 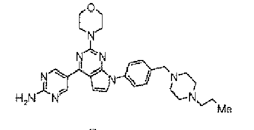 | single bond | 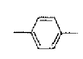 | 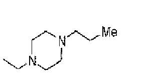 |
| 42 | 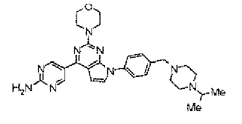 | single bond | 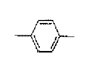 | 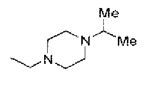 |
| 43 | 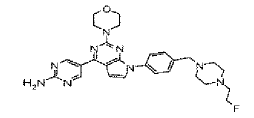 | single bond | 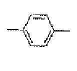 | 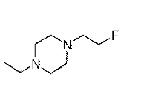 |
| 44 | 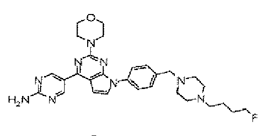 | single bond | 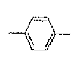 | 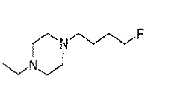 |
| 45 | 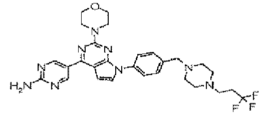 | single bond | 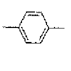 | 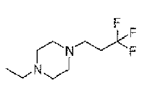 |
| 46 | 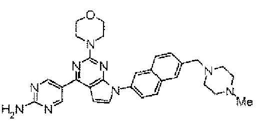 | single bond | 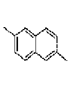 | 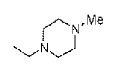 |
| 47 | 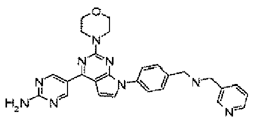 | single bond | 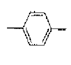 | 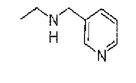 |

The compound of the present invention and a pharmaceutically acceptable salt thereof include all stereoisomers of the compound of the present invention represented by formula (I) (for example, enantiomers, diastereomers (including cis- and trans- geometrical isomers)), racemic forms of the aforemenetioned isomers and the other mixtures thereof. In particular, the compound represented by the formula (I) includes stereoisomers in the present invention.

In addition, several tautomeric forms such as enol and imine forms, keto and examine forms and mixtures thereof may exist for the compound of the present invention and a pharmaceutically acceptable salt thereof. Tautomers are present in solution as a mixture of tautomer set. In solid forms, one of the tautomers is usually dominant. Although one of the tautomers may be described, all tautomers of the compound of the present invention are included in the present invention.

Moreover, atropisomers of the present invention are also included in the present invention. Atropisomers refer to Compound I represented by the formula (I) capable of being separated into isomers having limited rotation.
These isomers can be separated by ordinary methods utilizing differences in physicochemical properties between isomers. For example, racemic compounds can be converted to stereochemically pure isomers using a typical optical resolution method such as optical resolution by deriving to a diastereomer salt with an optically active acid such as tartaric acid. Mixtures of diastereomers can be separated by using fractional crystallization or various types of chromatography (such as thin layer chromatography, column chromatography or gas chromatography).

In addition, the compound as claimed in the present invention, whether it be in a free form or in the form of a pharmaceutically acceptable salt, is included in the present invention. There are no particular limitations on this "salt" provided it forms a salt with the compound represented by formula (I) as claimed in the present invention (also referred to as Compound I) and is a pharmaceutically acceptable salt, and examples thereof include an acid salt formed by Compound I of the present invention and an acid, and a basic salt formed by Compound I of the present invention and a base.

The acid used to prepare a pharmaceutically acceptable acid salt of Compound I of the present invention is preferably that which reacts with Compound I of the present invention and forms a non-toxic acid salt. Examples of acid salts include hydrochlorides, hydrobromides, hydroiodides, nitrates, sulfates, bisulfates, phosphates, acid phosphates, acetates, lactates, citrates, acid citrates, tartrates, bitartrates, succinates, oxalates, malates, fumarates, gluconates, malonates, saccharates, benzoates, mandelates, salicylates, trifluoroacetates, propionates, glutarates, methane sulfonates, ethane sulfonates, benzene sulfonates, p-toluene sulfonates and 1,1'-methylene-bis-(2-hydroxy-3-naphthoates).

The base used to prepare a pharmaceutically acceptable basic salt of Compound I of the present invention is preferably that which reacts with Compound I of the present invention and forms a non-toxic basic salt. Examples of basic salts include alkaline metal salts such as sodium salts and potassium salts, alkaline earth metal salts such as calcium salts and magnesium salts, ammonium salts, water-soluble amine addition salts such as N-methylglucamine salts, lower alkanol ammonium salts, and salts derived from other pharmaceutically acceptable bases of organic amines.

In addition, since Compound I of the present invention may absorb moisture, become adhered with moisture and form a hydrate if allowed to stand in air, such salts are included in the present invention as salts of Compound I.

Moreover, although Compound I of the present invention may also absorb some types of solvents resulting in the formation of a solvate, such salts are also included in the present invention as salts of Compound I.

In the case of obtaining a compound of formula (I) as claimed in the present invention in a free form, the free form can be converted to a salt optionally formed by a compound of formula (I) or a hydrate or solvate thereof in accordance with ordinary methods.

In addition, in the case of obtaining a compound of formula (I) as claimed in the present invention in the form of a salt, hydrate or solvate of a compound of formula (I), that salt, hydrate or solvate can be converted to a free form of a compound of formula (I) in accordance with ordinary methods.

The compound represented by formula (I) 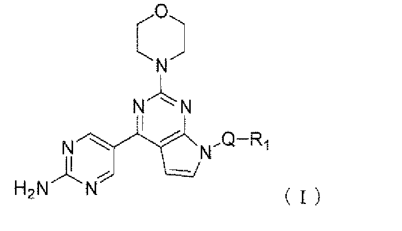 [wherein, Q and R₁ are the same as defined in formula (I)] of the present invention can be prepared by the process which comprises reacting the compound represented by formula (VIa): 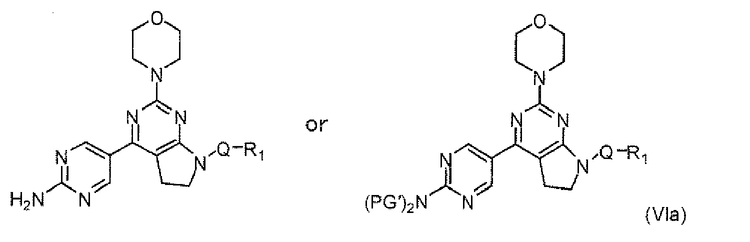 [wherein, Q and R₁ are the same as defined above; and PG' represents an amino group-protecting group]
with an oxidizing agent, and thereafter may further comprise removing the amino group-protecting group.

Furthermore, the compound used in the aforementioned process as a starting material and represented by formula (VIa): 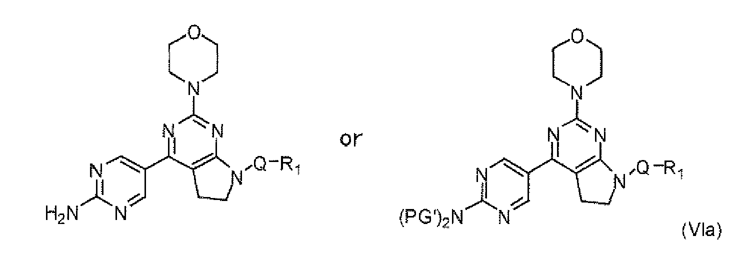 [wherein, Q and R₁ are the same as defined above; and PG' represents an amino group-protecting group]
can be prepared by the step of reacting the compound represented by formula (Va): 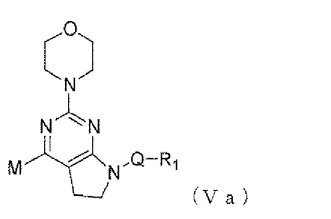 [wherein, M represents a leaving group; and Q and R₁ are the same as defined above] with a boronic acid derivative represented by the following formula: 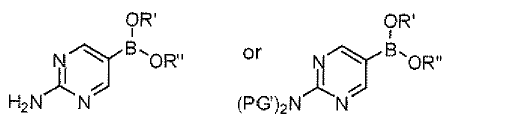 [wherein, R' and R" each independently represent a hydrogen atom or C₁₋₆ alkyl, or R' and R" may together form -C₂₋₃alkylene-, where -C₂₋₃ alkylene- may be substituted at 1 to 4 locations by -methyl; and PG' represents an amino group-protecting group] in the presence of a palladium catalyst and a ligand.

The compound of formula (I) of the present invention can be prepared from the compound represented by formula (Va) via the compound represented by formula (VIa) by continuously performing the aforementioned steps.

### Typical Process for Synthesizing Compound of Formula (I)

The following provides a specific explanation of the process for synthesizing the compound of formula (I) of the present invention. Although the compound of the present invention represented by formula (I) can be synthesized according to ordinary organic synthesis means such as the process indicated below, the synthesis process of the compound represented by formula (I) of the present invention is not limited thereto. Furthermore, in the synthesis process indicated below, in the case defined groups are subjected to undesirable chemical conversion under the conditions of the process used, synthesis can be carried out by using means such as protection and deprotection of functional groups unless specifically stated otherwise in the description. An example of a procedure for selecting as well as attaching and removing protecting groups is the method described in Greene and Wuts, "Protective Groups in Organic Synthesis" (3rd edition, Wiley-VCH, Inc., 1999), and these methods may be suitably used depending on the reaction conditions. In addition, the order of the reaction steps, such as the introduction of substituents, can be changed as necessary. In addition, in the synthesis process described below, a desired product can be obtained by carrying out a functional group modification reaction at a suitable stage in a series of reaction steps after having carried out the reaction with a raw material having a functional group serving as a precursor. The functional group modification reaction can be carried out by the method described in, for example, Smith and March, "March's Advanced Organic Chemistry" (5th edition, Wiley-VCH, Inc., 2001) or Richard C. Larock, "Comprehensive Organic Transformations" (VCH Publishers, Inc., 1989). Commercially available products may be used for the raw material compounds used during synthesis, or the raw material compounds may also be synthesized in accordance with ordinary methods as necessary.

In addition, compounds represented by general formula (I) described in the following reaction steps are compounds of the present invention represented by general formula (I) or said compounds in which substituents are protected with suitable protecting groups. Among the compounds represented by general formula (I), said compounds protected with a protecting group can be converted to the compounds of the present invention represented by general formula (I) by suitably going through a deprotection step in accordance with ordinary methods. In addition, protection steps and deprotection steps in accordance with ordinary methods are suitably included in the following reaction steps. Examples of groups used as protecting groups for the amino group include carbamate-based protecting groups such as a methoxycarbonyl, cyclopropylmethoxycarbonyl, ethoxycarbonyl, 2,2,2-trichloroethoxycarbonyl, 2-iodocthoxycarbonyl, 2-trimethylsilylethoxycarbonyl, 2-methylthioethoxycarbonyl, 2-methylsulfonylethoxycarbonyl, isobutyloxycarbonyl, t-butoxycarbonyl (BOC), 9-fluorenylmethoxycarbonyl (Fmoc), benzyloxycarbonyl (CBZ), p-methoxybenzyloxycarbonyl, 2,4-dichlorobenzyloxycarbonyl or p-cyanobenzyloxycarbonyl group; amide-based protecting groups such as formyl groups, acetyl groups, dichloroacetyl groups, trichloroacetyl groups, trifluoroacetyl groups, or benzoyl group (Bz); hydrocarbon chain-based protecting group such as methyl and allyl; and benzyl-based protecting groups such as benzyl groups, o-methoxybenzyl groups (2-methoxybenzyl group), m-methoxybenzyl groups (3-methoxybenzyl group), p-methoxybenzyl groups (4-methoxybenzyl group or PMB group), o,m-dimethoxybenzyl groups (2,3-dimethoxybenzyl group), o,p-dimethoxybenzyl groups (2,4-dimethoxybenzyl group), m,p-dimethoxybenzyl groups (3,4-dimethoxybenzyl group), and o,m,p-trimethoxybenzyl groups (2,3,4-trimethoxybenzyl group). Among these, benzyl-based protecting groups are preferably mentioned as a PG of the amino group to be described later; as PG', benzyl-based protecting groups are preferably mentioned; as PG", amide-based protecting groups are preferably mentioned.

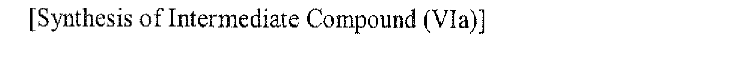

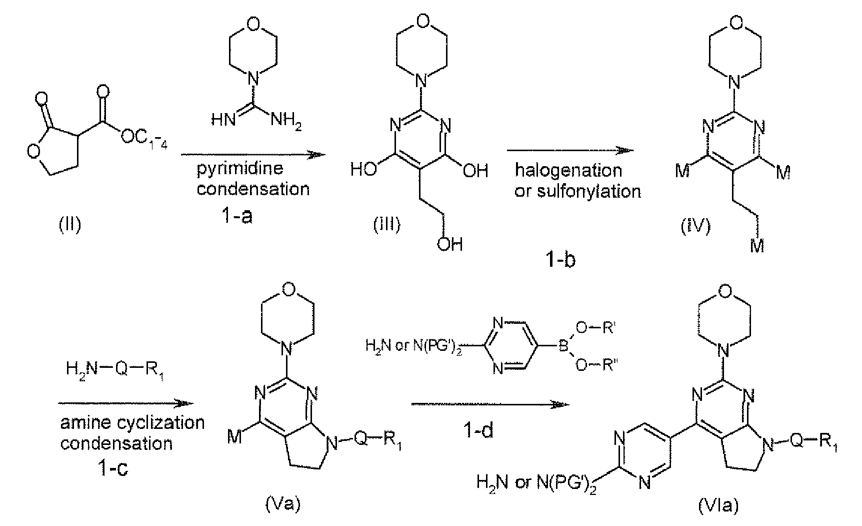

[wherein, -OC₁₋₄ represents -C₁₋₄ alkyloxy (preferably -methoxy), R' and R" are each independently a hydrogen atom or C₁₋₆ alkyl, or R' and R" may together form -C₂₋₃alkylene-, where -C₂₋₃alkylene- may be substituted at 1 to 4 locations by -methyl (preferably -(1,1,2,2-tetramethyl-ethylene)-); PG' represents an amino group-protecting group (preferably PMB); M denotes a leaving group, preferably a halogen atom such as a chlorine atom, bromine atom, or iodine atom, an alkylsulfonyloxy group such as methanesulfonyloxy, an arylsulfonyloxy group such as - toluenesulfonyloxy, or a halogenoalkylsulfonyloxy group such as - trifluorotnethanesulfonyloxy, more preferably a chlorine atom. Q and R₁ are the same as previously defined.]

The present synthesis process comprises converting a triol derivative, which is obtained by condensing 3-C₁₋₄ alkoxycarbonyl-γ-lactone and morpholinoformamidine, to a trihalogen form or trisulfonic acid ester form (preferably trichloro form), followed by cyclization and condensation with a primary amine having a desired group (H₂N-Q-R₁), and a coupling reaction with a boronic acid derivative to obtain an intermediate compound (VIa).

3-C₁₋₄ alkoxycarbonyl-γ-lactone (II) can be prepared by a known process (for example, it can be synthesized according to a process described in J. Org. Chem. (1978), 43(2), 346-347).

Step 1-a is a step of synthesizing a triol derivative (III) by condensation reaction, in an inert solvent and in the presence of base, of 3-C₁₋₄ alkoxycarbonyl-γ-lactone (II) and morpholinoformamidine (preferably, morpholinoformamidine chlorohydric acid salt, or morpholinoformamidine hydrobromic acid salt (Alfa Aesar GmbH & Co. KG, etc.)) (references: D. L. Dunn et al., J. Org. Chem. Vol. 40, p. 3713, 1975; K. Burdeska et al., Helv. Chim. Acta, Vol. 64, p. 113, 1981; P. Wang et al., Huaxue Xuebao, Vol. 42, p. 722, 1984). Examples of the inert solvent include methanol, ethanol, t-butanol, tetrahydrofuran, dimethoxy ethanol, and 1,4-dioxane; and examples of the base include sodium methoxide, sodium ethoxide, potassium methoxide, potassium ethoxide, potassium t-butoxide, and triethylamine. The reaction temperature is, for example, room temperature to the boiling point of the solvent (the boiling point of the solvent refers to carrying out a reaction under heat reflux condition), preferably, room temperature to 100°C, and the reaction time is, for example, 30 minutes to 12 hours.

Step 1-b is a process for synthesizing trihalogen derivative or trisulfonic acid ester derivative (IV) by halogenation or sulfonylation of triol derivative (III) in an inert solvent or in the absence of solvent and in the presence of a halogenation agent or sulfonylation agent. Examples of the inert solvent include dimethylformamide, dimethylacetamide, N-methylpyrrolidone, toluene, xylene, acetonitrile and dichloromethane; and examples of halogenation agents include phoshporus oxychloride, thionyl chloride and Vilsmeier reagent (J. C. S. Perkin I (1976) 754-757). An amine base or a hydrochloride thereof such as triethylamine or diisopropylethylamine, or a quaternary ammonium salt such as tetraethylammonium chloride, n-tetrabutylammonium chloride may coexist with a halogenation agent. Examples of sulfonylation agents include methanesulfonyl chloride, toluenesulfonyl chloride, trifluoromethanesulfonic acid anhydride. An amine base such as triethylamine or diisopropylethylamine may coexist with a sulfonylation agent. The reaction temperature is, for example, room temperature to the boiling point of a solvent or reagent (the boiling point of the solvent refers to carrying out a reaction under heat reflux condition), preferably, room temperature to 200°C, and the reaction time is, for example, 30 minutes to 20 hours (references: A. Gangjee et al., J. Med. Chem. Vol. 43, p. 3837, 2000; P. Rajamanickam et al., Indian J. Chem. Section B: Vol. 26B, p. 910, 1987).

Step 1-c is a reaction to obtain an intermediate compound (Va) by cyclization condensation reaction of trihalogen derivative or trisulfonic acid ester derivative (IV) with a primary amine derivative (H₂N-Q-R₁) in an inert solvent and in the presence of base. Examples of the inert solvent include dimethylformamide, dimethylacetamide, N-methylpyrrolidone (NMP), toluene, 1,4-dioxane, dimethoxyethane, and acetonitrile. Examples of the base include potassium carbonate, cesium carbonate, sodium hydroxide, potassium t-butoxide, sodium hydride, potassium phosphate (tripotassium phosphate), lithium bistrimethylsilylamide (LiN(TMS)₂) (references: E. Bisagni et al., J. Org. Chem. Vo. 47, p. 1500, 1982). The reaction in step 1-c may suitably be performed in the presence of a palladium catalyst or a ligand. Examples of such palladium catalyst include PdCl₂, Pd(OH)₂, Pd(OAc)₂, Pd₂dba₃, PdCl₂[P(o-tol)₃]₂, PdCl₂(PPh₃)₂, and Pd(O₂CCF₃)₂, and examples of such ligands include PPh₃, P(o-tol)₃, P(t-Bu)₃, dppf, BINAP, 2',6'-dimethoxy-2-(dicyclohexylphosphino)biphenyl (S-Phos), 2-dicyclohexylphosphino-2',4',6'-triisopropylbiphenyl (X-Phos), 4,5-bis(diphenylphosphino)-9,9-dimethylxanthene (Xantphos), and 1,3-bis(2,6-diisopropylphenyl)imidazol-2- ylidene.
The reaction temperature is, for example, from room temperature to the boiling point of the solvent or reagent (the boiling point of the solvent refers to carrying out a reaction under heat reflux condition), and the reaction time is, for example, 30 minutes to 20 hours.

Step 1-d is a reaction to obtain an intermediate compound (VIa) by a coupling reaction (for example, Suzuki reaction) of an intermediate compound (Va) with a boronic acid derivative (preferably, 5-(4,4,5,5-tetramethyl-[1,3,2]dioxaborolan-2-yl)-pyrimidin-2-ylamine or bis-(4-methoxybenzyl)-[5-(4,4,5,5-tetramethyl-[1,3,2]dioxaborolan-2-yl)pyrimidin-2-yl]amine) in an inert solvent and in the presence of base, palladium catalyst, or suitable ligand (references: M. Havelkova et al., Synlett, p. 1145, 1999; G. Luo et al., Tetrahedron Lett. Vol. 43, p. 5739, 2002).
Examples of the inert solvent include dimethylformamide, dimethylacetamide, N-methylpyrrolidone, toluene, tetrahydrofuran, 1,4-dioxane, and dimethoxyethane, which may contain 1% to 50% of water; examples of the palladium catalyst include PdCl₂, Pd(OH)₂, Pd(OAc)₂, Pd₂dba₃, PdCl₂[P(o-tol)₃]₂, Pd(O₂CCF₃)₂, and PdCl₂(PPh₃)₂; examples of the ligand include PPh₃, P(o-tol)₃, P(t-Bu)₃, dppf, BINAP, 2',6'-dimethoxy-2-(dicyclohexylphosphino)biphenyl (S-Phos), 2-dicyclohexylphosphino-2',4',6'-triisopropylbiphenyl (X-Phos), 4,5-bis(diphenylphosphino)-9,9-dimethylxanthene (Xantphos), and 1,3-bis (2,6-diisopropylphenyl)imidazol-2-ylidene; and examples of the base include sodium carbonate, potassium carbonate, cesium carbonate, sodium hydroxide, potassium t-butoxide, potassium phosphate, and lithium bis-trimethylsilylamide (LiN(TMS)₂). The reaction temperature is, for example, 0 to 110°C, and preferably 25 to 110°C, and the reaction time is, for example, 30 minutes to 20 hours. In addition, the intermediate compound (VIa) can be synthesized by using instead of boronic acid an arylzinc compound prepared by a known method (Metal-Catalyzed Cross-Coupling Reactions, 2nd ed., 2004, Vol. 2, p. 815).
Furthermore, the subsequent removal of PG' (deprotection) may be or may not be carried out. As a deprotection method, for example, when PG' is a benzyl-based protecting group, the same reaction as the one for removing the benzyl-based protecting group to be described later in step 2-b may be carried out.

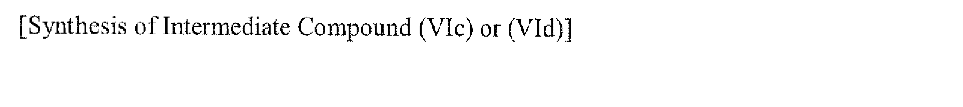

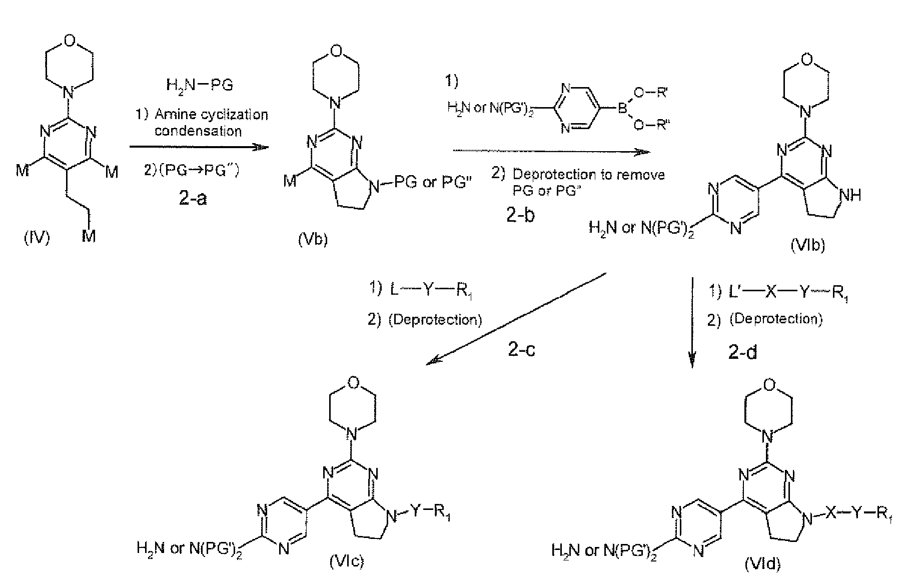

[wherein, PG, PG' and PG" represent an amino group-protecting group; L and L' represent a leaving group; and M, R', R", X, Y, Q and R₁ are the same as previously defined].

This synthesis step shows a synthesis process of an intermediate compound (VIc) when, in particular, Q is -Y- (when X is a single bond), and a synthesis process of an intermediate compound (VId) when Q is -X-Y- (preferably, Y is a single bond), in the intermediate represented by formula (VIa).
Step 2-a is a reaction to obtain an intermediate compound (Vb), by cyclization condensation reaction of a trihalogen derivative or a trisulfonic acid ester derivative (IV) with a primary amine derivative (H₂N-PO) in an inert solvent and in the presence of base, followed by suitable replacement of the amine protecting group (PG to PG"). In the cyclization condensation of this step, the reaction can be carried out in the same manner as Step 1-c. Examples ofPGs here include amine protecting groups, for example, carbamate-based protecting groups such as methoxy carbonyl, ethoxycarbonyl, t-butoxycarbonyl, benzyloxycarbonyl or 9-fluorenylmethoxycarbonyl (Fmoc); amide-based protecting groups such as a formyl, acetyl, chloroacetyl, trichloroacetyl, trifluoroacetyl or benzoyl group; hydrocarbon chain-based protecting groups such as a methyl or allyl group; and benzyl-based protecting groups such as a benzyl, 4-methoxybenzyl or 2,4-dimethoxybenzyl group, preferably benzyl-based protecting groups, more preferably 2,4-dimethoxybenzyl and 4-methoxybenzyl. Examples of NH₂PG include 4-methoxybenzylamine and 2,4-dimethoxybenzylamine (Aldrich, etc.).
Replacement of the amine protecting group (PG to PG") may be carried out after the cyclization condensation reaction. Examples ofPG" here as the amine protecting group include similar protecting groups to PG, preferably amide-based protecting groups, more preferably acetyl.
In replacement of the amine protecting group (PG to PG") after cyclization condensation reaction, indicated below is an example of deprotection reaction for removing PG when PG in NH₂PG is a benzyl-based protecting group (preferably 2,4-dimethoxybenzyl or 4-methoxybenzyl). A compound obtained after cyclization condensation reaction can be deprotected by removing PG by carrying out a reaction, for example, in an inert solvent or in the absence of solvent and in the presence of acid. Examples of the inert solvent include dichloromethane and ethyl acetate. Examples of the acid include trifluoroacetic acid, sulfuric acid, hydrochloric acid, formic acid and acetic acid, and two different types of acids may be used. Trifluoroacetic acid or sulfuric acid are preferred. A preferable deprotection method is the method of treating with trifluoroacetic acid in the absence of solvent or the method of using ethyl acetate and sulfuric acid. N-acetylcysteine in an amount equal to or greater than the equivalent amount of the reactant (an intermediate compound (Vb)) may be used together. The reaction temperature is normally 0 to 120°C, preferably room temperature to 80°C. The reaction time is, for example, 30 minutes to 12 hours. Furthermore, removal of PG (deprotection) can be carried out also by a method comprising treating by catalytic hydrogenation using palladium carbon and the like.

Then, an example of a reaction comprising removing PG (deprotection) followed by re-protection with PG" will be shown below. When PG" is, for example, amide-based, such reaction can be carried out in an inert solvent and in the presence or absence of base, by means of acid halide method, acid anhydride method, active esterification method or condensation method. Preferably, acid halide method or acid anhydride method can be employed. Examples of inert solvents in acid halide method includes dichloromethane, tetrahydrofuran, dioxane, diethyl ether, dimethoxyethane, acetone, acetonitrile, dimethylformamide, dimethylacetamide, dimethylsulfoxide, toluene, and benzene; preferably dichloromethane, tetrahydrofuran, dimethoxyethane, dimethylformamide, and acetonitrile. As the acid halide, preferably acetyl chloride can be mentioned. In the reaction, a base may be present and examples of such base include triethylamine, diisopropylethylamine, pyridine, dimethylaminopyridine, potassium hydride, sodium hydride, potassium bis-trimethylsilylamide, sodium bis-trimethylsilylamide, sodium metal, potassium carbonate, cesium carbonate, lithium bis-trimethylsilylamide, and lithium diisopropylamide, and preferably triethylamine, diisopropylethylamine, pyridine, dimethylaminopyridine, potassium carbonate, or cesium carbonate.
Examples of inert solvents in acid anhydride method include dichloromethane, tetrahydrofuran, dioxane, diethyl ether, dimethoxyethane, acetone, acetonitrile, dimethylformamide, dimethylacetamide, dimethylsulfoxide, toluene, benzene, or no solvent, preferably dichloromethane, tetrahydrofuran, dimethoxyethane, dimethylformamide, acetonitrile, or no solvent. As acid anhydride, preferably acetic anhydride can be mentioned. The reaction can be carried out in the presence of base, and examples of such bases include triethylamine, diisopropylethylamine, pyridine, dimethylaminopyridine, potassium hydride, sodium hydride, potassium bistrimethylsilylamide, sodium bistrimethylsilylamide, sodium metal, potassium carbonate, cesium carbonate, lithium bis-trimethylsilylamide, and lithium diisopropylamide, and preferably triethylamine, diisopropylethylamine, pyridine, dimethylaminopyridine, potassium carbonate, and cesium carbonate.

Step 2-b is a reaction to obtain an intermediate compound (VIb) by a coupling reaction (for example, Suzuki reaction) of an intermediate compound (Vb) with a boronic acid derivative (preferably, 5-(4,4,5,5-tetramethyl-[1,3,2]dioxaborolan-2-yl)-pyrimidin-2-yl]amine or bis-(4-methoxybenzyl)-[5-(4,4,5,5-tetramethyl-[1,3,2]dioxaborolan-2-yl)-pyrimidin-2-yl]amine) in an inert solvent and in the presence of a base, palladium catalyst, or suitable ligand.
The reaction of an intermediate compound (Vb) and a boronic acid derivative can be carried out in the same manner as step 1-d.
With respect to the subsequent deprotection step, when PG or PG" is, for example a benzyl-based protecting group (preferably 2,4-dimethoxybenzyl or 4-methoxybenzyl), deprotection can be carried out by the same method as the aforementioned method. In addition, when PG or PG" is, for example an amide-based protecting group (preferably acetyl), deprotection can be carried out by reacting the compound intended to be deprotected in an inert solvent and in the presence of base. Examples of the inert solvent include methanol, ethanol, tetrahydrofuran, and water, and examples of the base include sodium hydroxide, lithium hydroxide, and sodium carbonate. The reaction temperature is 0 to 120°C, preferably room temperature to 100°C, and the reaction time is, for example, 30 minutes to 15 hours.

Step 2-c is a synthesis process of an intermediate compound (VIc) when Q is, in particular, -Y- (X is a single bond). The intermediate compound (VIc) can be synthesized by reacting an intermediate compound (VIb) with L-Y-R₁ (wherein L denotes a leaving group, preferably a halogen atom such as a chlorine atom, bromine atom, or iodine atom, or trifluoromethanesulfonyloxy, and more preferably bromine atom) in an inert solvent and in the presence or absence of base, in the presence of a palladium catalyst or suitable ligand, followed by suitable removal of PG' (deprotection). This reaction can be carried out by referring, for example, to a reaction for introducing a cyclic group by a coupling reaction with a known halogenated cyclic group (Org. Lett., Vol. 2, p. 1101, 2000; Tetrahedron Lett., Vol. 42, p. 7155, 2001).
Examples of the inert solvent include tetrahydrofuran, dioxane, diethyl ether, dimethoxyethane, dimethylformamide, dimethylacetamide, dimethylsulfoxide, acetone, acetonitrile, toluene, and benzene, while preferable examples include toluene, 1,4-dioxane, dimethoxyethane, tetrahydrofuran, and dimethylformamide. Examples of the palladium catalyst include PdCl₂, Pd(OAc)₂, Pd₂dba₃, PdCl₂(PPh₃)₂, PdCl₂[P(o-tol)₃]₂, Pd(O₂CCF₃)₂, palladium carbon, palladium black, and Pd(OH)₂, while preferable examples include PdCl₂, Pd(OAc)₂, Pd₂dba₃, PdCl₂[P(o-tol)₃]₂, Pd(O₂CCF₃)₂, and PdCl₂(PPh₃)₂. Examples of the ligand include triphenylphosphine, P(o-tol)₃, BINAP, DPPF, P(t-Bu)₃, 2-dicyclohexylphosphino-2'-(N,N-dimethylamino)biphenyl, 2-(di-t-butylphosphino)biphenyl, 2-(dicyclohexylphosphino)biphenyl, 2',6'-dimethoxy-2-(dicyclohexylphosphino)biphenyl (S-Phos), 2-dicyclohexylphosphino-2,4',6'-triisopropylbiphenyl (X-Phos), 2',4',6'-triisopropyl-2-(dicyclohexylphosphino)biphenyl, 4,5-bis(diphenylphosphanyl)-9,9-dimethyl-9H-xanthene (Xantphos), 4,5-bis[bis(3,5-bistrifluoromethylphenyl)phosphanyl]-9,9-dimethyl-9H-xanthene, 1,3-diallyldihydroimidazolium salt, 2,8,9-triisobutyl-2,5,8,9-tetraaza-1-phospha-bicyclo[3.3.3]undecane, and 1,3-bis (2,6-diisopropylphenyl)imidazol-2-ylidene, preferably triphenylphosphine, BINAP, 2',6'-dimethoxy-2-(dicyclohexylphosphino)biphenyl, 2' ,4' ,6' -triisopropyl-2-(dicyclohexylphosphino)biphenyl, and 2,8,9-triisobutyl-2,5,8,9-tetraaza-1-phosphabicyclo[3.3.3]undecane. Examples of the base include sodium hydroxide, potassium hydroxide, sodium methoxide, sodium ethoxide, potassium bis-trimethylsilylamide, sodium bis-trimethylsilylamide, lithium bis-trimethylsilylamide (LiN(TMS)₂), lithium diisopropylamide, cesium carbonate, sodium t-butoxide, potassium t-butoxide, and potassium phosphate, while preferable examples include cesium carbonate, sodium hydroxide, sodium t-butoxide, potassium t-butoxide, potassium phosphate, and lithium bis-trimethylsilylamide.
Although varying according to the types of solvent and base and the like, the reaction temperature is, for example, 0°C to the boiling point of the solvent (the boiling point of the solvent refers to carrying out a reaction under heat reflux condition), and preferably room temperature to the boiling point of the solvent. Furthermore, although varying according to the reaction temperature and the like, the reaction time is normally 30 minutes to 100 hours and preferably 30 minutes to 24 hours.

The subsequent removal of PG' (deprotection) may be or may not be carried out. As a deprotection method, for example, when PG' is a benzyl-based protecting group, the same reaction as the one for removing a benzyl-based protecting group described in step 2-b may be carried out.

It should be noted that, when PG' deprotection is carried out in step 2-c after the coupling reaction with L-Y-R₁, (Step 2-c' in the following formula), the oxidation reaction from pyrrolidine ring to pyrrole ring may occur simultaneously with deprotection reaction for removing PG' to give a compound represented by formula (I'): 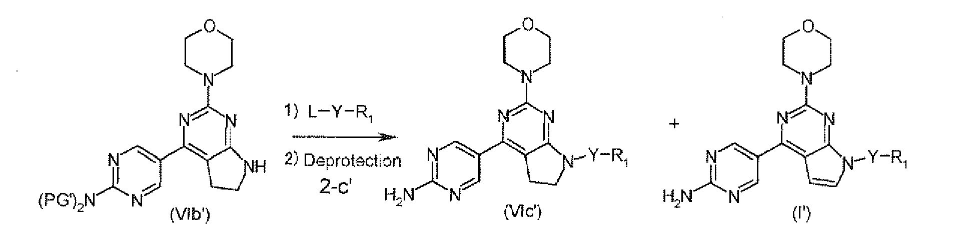 [wherein, PG', L, Y, R₁ are the same as previously defined].
Such deprotection conditions for removing PG' include, for example, the case where, when PG' is 4-methoxybenzyl, a solvent amount of trifluoroacetic acid and further N-acetylcysteine in an amount equal to or greater than the equivalent amount of the intermediate compound (VIb') are used together.

Step 2-d is a synthesis process of an intermediate compound (VId) when Q is -X-Y-. The intermediate compound (VId) can be synthesized, wherein -X- is, in particular, -CO-, by reacting an intermediate compound (VIb) and carboxylic acid or a carboxylic acid reactive derivative represented by L'-CO-Y-R₁ (wherein L' represents a leaving group, preferably a halogen atom such as a chlorine atom, bromine atom, iodine atom; or -OH, -OC₁₋₆ alkyl, more preferably a chlorine atom or bromine atom) in an inert solvent and in the presence or absence of base, by means of acid halide method, acid anhydride method, active esterification method or condensation method, suitably followed by removal of a protecting group, PG' (deprotection). Removal of PG' (deprotection) will be described later.
The acid halide method is achieved by synthesizing an acid halide (Hal-Y-R₁, Hal-CO-Y-R₁, Hal-CONH-Y-R₁, Hal-CON(C₁₋₄ alkyl)-Y-R₁, Hal-CS-Y-R₁, Hal-CSNH-Y-R₁, Hal-CSN(C₁₋₄ alkyl)-Y-R₁, or Hal-SO₂-Y-R₁ ; as such Hal, for example, a chlorine atom and bromine atom may be mentioned) by reacting an acid having a desired group with a halogenation agent in an inert solvent or in the absence of solvent and then reacting this acid halide with an intermediate compound (VIb) in an inert solvent. This reaction may be carried out in the presence of base.
Examples of the halogenation agent include thionyl chloride, oxalic chloride and phosphorous pentachloride.
Examples of the inert solvent used include dichloromethane, tetrahydrofuran, dioxane, diethyl ether, dimethoxyethane, acetone, acetonitrile, dimethylformamide, dimethylacetamide, dimethylsulfoxide, toluene and benzene, while preferable examples include dichloromethane, tetrahydrofuran, dimethoxyethane, dimethylformamide and acetonitrile.
Examples of the base used include triethylamine, diisopropylethylamine, pyridine, dimethylaminopyridine, potassium hydride, sodium hydride, potassium bis-trimethylsilylamide, sodium bis-trimethylsilylamide, sodium metal, potassium carbonate, cesium carbonate, lithium bis-trimethylsilylamide, and lithium diisopropylamide, while preferable examples include triethylamine, diisopropyl ethylamine, pyridine, dimethylaminopyridine, potassium carbonate and cesium carbonate.
Although the reaction temperature varies according to the types of solvent and base and the like, in the synthesis of an acid halide by the the reaction with a halogenation agent and in the reaction between an acid halide and an intermediate compound (VIb), each reaction can be carried out, for example, -20°C to the boiling point of the solvent (the boiling point of the solvent refers to carrying out a reaction under heat reflux condition), and preferably room temperature to the boiling point of the solvent. Although varying according to the reaction temperature and the like, the reaction time is 15 minutes to 100 hours and preferably 30 minutes to 24 hours.

The mixed acid anhydride method is achieved by reacting a C₁₋₆ alkyl halogenoformate or C₁₋₆ alkylcarboxylic anhydride (wherein, the C₁₋₆ alkyl represents a linear or branched alkyl group having 1 to 6 carbon atoms) with a carboxylic acid having a desired group (HOOC-Y-R₁) to synthesize a mixed acid anhydride (C₁₋₆ alkyl OOC-Y-R₁) followed by reacting the mixed acid anhydride and an intermediate compound (VIb). The reaction for synthesizing the mixed acid anhydride is carried out by reacting a compound including a C₁₋₆ alkyl halogenocarbonate such as methyl chlorocarbonate, ethyl chlorocarbonate, isobutyl chlorocarbonate or hexyl chlorocarbonate (preferably ethyl chlorocarbonate or isobutyl chlorocarbonate), a C₁₋₆ alkyl carboxylic anhydride such as acetic anhydride or propionic anhydride (preferably acetic anhydride), and is preferably carried out in an inert solvent in the presence of base.
The same bases and inert solvents used in the acid halide method of this step are used for the base and inert solvent. Although varying according to the type of solvent and the like, the reaction temperature is normally -20 to 50°C (preferably 0 to 30°C). Although varying according to the reaction temperature and the like, the reaction time is normally 15 minutes to 24 hours (and preferably 30 minutes to 15 hours).

The condensation method is carried out by directly reacting an intermediate compound (VIb) with a carboxylic acid (HOOC-Y-R₁) having a desired group in an inert solvent, in the presence of a condensation agent and in the presence or absence of base (preferably in the presence of base).
Examples of the inert solvent used include dichloromethane, tetrahydrofuran, dioxane, diethyl ether, dimethoxyethane, dimethylformamide, dimethylacetamide, dimethylsulfoxide, acetone, acetonitrile, toluene and benzene, while preferable examples include dichloromethane, tetrahydrofuran, dimethoxyethane, dimethylformamide and acetonitrile.

In addition, examples of the condensation agent used include 1,3-dicyclohexylcarbodiimide (DCC), 2-ethoxy-1-ethoxycarbonyl-1,2-dihydroquinoline (EEDQ), bromo-tris(pyrrolidino)-phosphonium hexafluorophosphate (PyBrOP), 1-ethyl-3-(3'-dimethylaminopropyl)carbodiimide hydrochloride (WSCI) or (benzotriazolyloxy)tripyrrolidino-phosphonium hexafluorophosphate (PyBOP), 3-hydroxy-4-oxo-3,4-dihydro-1,2,3-benzotriazine (HODhBt) and hydroxybenzotriazole (HOBt). In addition, other examples include the combination of 1-ethyl-3-(3'-dimethylaminopropyl)carbodiimide (EDC) and N-hydroxybenzotriazole (HOBt) and the combination of 1-ethyl-3-(3'-dimethylaminopropyl)carbodiimide hydrochloride (WSCI) and 3-hydroxy-4-oxo-3,4-dihydro-1,2,3-benzotriazine (HODhBt).

In addition, examples of the base used include diisopropylethylamine, triethylamine, pyridine, dimethylaminopyridine, potassium hydride, sodium hydride, potassium bistrimethylsilylamide, sodium bistrimethylsilylamide, sodium metal, potassium carbonate, cesium carbonate, lithium bistrimethylsilylamide, and lithium diisopropylamide, while preferable examples include diisopropylethylamine, triethylamine, pyridine, potassium carbonate, cesium carbonate, and sodium hydride.

Although varying according to the types of solvent and base and the like, the reaction temperature is, for example, 0°C to the boiling point of the solvent (the boiling point of the solvent refers to carrying out a reaction under heat reflux condition) and preferably room temperature to the boiling point of the solvent. Although varying according to the reaction temperature and the like, the reaction time is 30 minutes to 15 hours.

Then, Step 2-d when, in particular, X is -SO₂- will be explained. Referring to a known sulfonylation method, an intermediate compound (VId) can be obtained by reacting an intermediate compound (VIb) and a desired sulfonylation agent in an inert solvent and in the presence of base, followed by suitable removal of PG' (deprotection) (M. Loegers et al., J. Am. Chem Soc. Vol. 117, p. 9139, 1995; H. Tanaka et al., Bull. Chem. Soc. Jpn. Vol. 61, p. 310, 1988.; J.-F.Rousseau et al., Heterocycles, Vol. 55, p. 2289, 2001).
Examples of the inert solvent include dichloromethane, tetrahydrofuran, dioxane, diethyl ether, dimethoxyethane, dimethylformamide, dimethylacetamide, dimethylsulfoxide, acetone, acetonitrile, toluene, and benzene, while preferable examples include dichloromethane, tetrahydrofuran, dimethoxyethane, dimethylformamide, and acetonitrile.
Examples of the base include potassium hydride, sodium hydride, potassium bis-trimethylsilylamide, sodium bis-trimethylsilylamide, sodium metal, lithium bis-trimethylsilylamide, lithium diisopropylamide, triethylamine, potassium carbonate, and cesium carbonate, while preferable examples include triethylamine, potassium carbonate, cesium carbonate, and sodium hydride.
Examples of such desired sulfonylation agent include sulfonic acid chloride (Cl-SO₂-Y-R₁), sulfonic acid anhydride (R₁-Y-SO₂-O-SO₂-Y-R₁), and sulfamoyl ester (C₁₋₆ alkyl O-SO₂-Y-R₁) having a desired group, while a preferable example is sulfonic acid chloride.
Although varying according to the types of solvent and base and the like, the reaction temperature is, for example, 0°C to the boiling point of the solvent (the boiling point of the solvent refers to carrying out a reaction under heat reflux condition), preferably room temperature to the boiling point of the solvent. Although varying according to the reaction temperature and the like, the reaction time is normally 30 minutes to 48 hours and preferably 30 minutes to 15 hours.
With respect to the subsequent deprotection reaction for removing PG', when PG' is, for example, a benzyl-based protecting group (preferably 2,4-dimethoxybenzyl or 4-methoxybenzyl), the same reaction as the one for removing a benzyl-based protecting group which is described in step 2-b can be carried out. In addition, instead of PG' deprotection, the following step may be carried out.

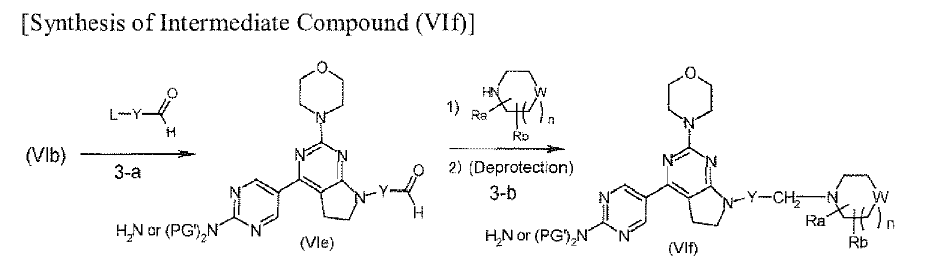

(wherein, Y, PG', L, W, n, Ra, R_{b} are the same as previously defined).
Among the intermediate compounds (VIc), the intermediate compound (VIf) wherein R₁ is, in particular, -CH₂-(heterocycle), can be synthesized through two steps from the intermediate compound (VIb) as previously described.

In step 3-a, an intermediate compound (VIe) can be synthesized by carrying out the same reaction as in step 2-c by using, instead of L-Y-R₁ in step 2-c, L-Y-CHO (Y and L are the same as previously defined, and preferably a halogen atom such as chlorine atom, bromine atom, or iodine atom) or an equivalent thereof (one in which the formyl group is protected).
Examples of equivalents to L-Y-CHO wherein the formyl group is protected include one in which the formyl group is acetalized. Specific examples include acyclic acetals such as dimethylacetal and diethyl acetal; and cyclic acetals such as 1,3-dioxane and 1,3-dioxolane. When performing step 3-a using a compound in which the formyl group is protected, a deprotection step for recovering the formyl group is necessary after the coupling with an intermediate compound (VIb). As a reaction, for example, of deprotecting acetal, a reaction can be carried out in an inert solvent and in the presence of acid.
Examples of the inert solvent include a lower alkyl alcohol such as methanol and ethanol, acetone, THF, dioxane, and water.
Examples of the acid include sulfuric acid, p-toluene sulfonic acid, trifluoroacetic acid, and hydrochloric acid, while preferable examples include sulfuric acid and hydrochloric acid.
Although varying according to the types of solvent and base and the like, the reaction temperature is, for example, 0°C to the boiling point of the solvent (the boiling point of the solvent refers to carrying out a reaction under heat reflux condition), and preferably 0 to 60°C. Furthermore, although varying according to the reaction temperature and the like, the reaction time is normally 30 minutes to 24 hours and preferably 30 minutes to 12 hours.

Step 3-b is a process for synthesizing an intermediate compound (VIf) by subjecting an intermediate compound (VIe) and a heterocyclic compound represented by the following formula: 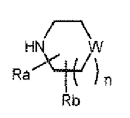 (wherein, W, Ra, R_{b}, and n are the same as previously defined) to a coupling reaction by means of a reductive amination reaction or the like in an inert solvent and in the presence of a hydride reducing agent (a reaction causing a reductive amination to obtain a corresponding amine). Then, a deprotection reaction may suitably be carried out.
Examples of the inert solvent include, methanol, ethanol, dichloromethane, THF, and dioxane.
Examples of the hydride reducing agent include sodium cyanoborohydride and sodium triacetoxyborohydride, and a preferable example is sodium triacetoxyborohydride.
The reaction temperature is, for example, -20°C to the boiling point of the solvent (the boiling point of the solvent refers to carrying out a reaction under heat reflux condition), and preferably 0 to 60°C. The reaction time is 30 minutes to 12 hours.
With respect to the deprotection reaction for removing PG', when PG' is, for example, a benzyl-based protecting group (preferably 2,4-dimethoxybenzyl or 4-methoxybenzyl), the same reaction for removing a benzyl-based protecting group which is described in step 2-b can be carried out. In addition, instead of PG' deprotection, the following step may be carried out.

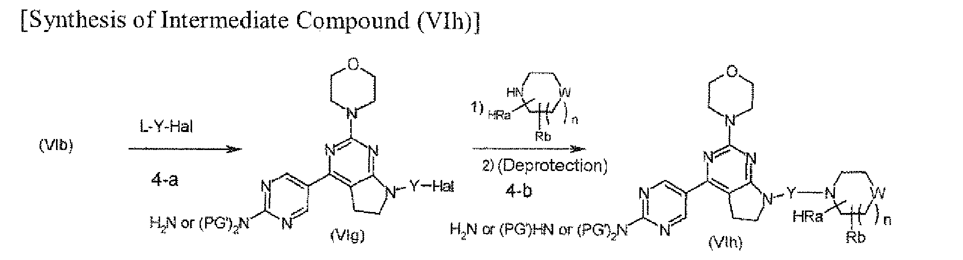

[wherein, PG', L (preferably an iodine atom), Hal (preferably chloro), Y, Ra, R_{b}, n and W are the same as previously defined.]
Among the intermediate compounds (VIa), in particular, the intermediate compound (VIh) wherein X in Q is a single bond can be synthesized through two steps from the intermediate compound (VIb) as previously described. When using this step, Y is preferably heteroarylene.
Steps 4-a and 4-b can be carried out in the same manner as the aforementioned step 2-c.
The subsequent deprotection for removing PG' may be or may not be carried out. As a deprotection method, for example, when PG' is a benzyl-based protecting group, the same reaction as the one for removing a benzyl-based protecting group described in step 2-b may be carried out.

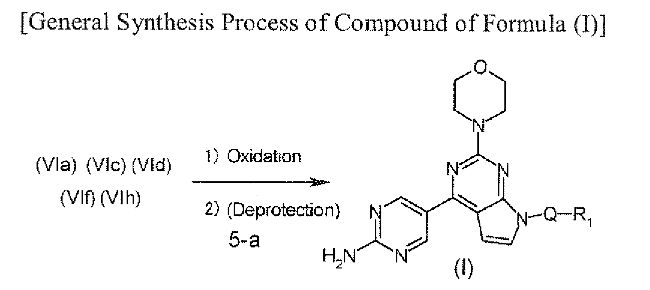

(wherein, Q and R₁ are the same as previously defined).
Step 5-a is a process for synthesizing a compound represented by formula (I) by an oxidation reaction of an intermediate compound (VIa), (VIc), (VId), (VIf) or (VIh) in an inert solvent and in the presence of an oxidizing agent (Heterocycles, 22(2), 379-86; 1984), followed by a suitable deprotection.
Examples of the oxidizing agent in the oxidation reaction include oxygen, 2,3,5,6-tetrachloro-1,4-benzoquinone, 2,3-dichloro-5,6-dicyano-1,4-benzoquinone (DDQ), manganese dioxide, selenium dioxide, and ceric ammonium nitrate (IV), and preferable examples include 2,3-dichloro-5,6-dicyano-1,4-benzoquinone.
Examples of the inert solvent used include methanol, ethanol, dichloromethane, chloroform, tetrahydrofuran, dioxane, diethyl ether, dimethoxyethane, dimethylformamide, dimethylacetamide, dimethylsulfoxide, acetone, acetonitrile, toluene, benzene, and water, while preferable examples include dichloromethane, chloroform, tetrahydrofuran, dimethylformamide, acetonitrile, and toluene. Furthermore, a mixed solvent may be used as a solvent, examples of which include a mixed solvent of dichloromethane and dimethylformamide (mixing ratio of, for example, 9:1 1 to 1:1), a mixed solvent of dichloromethane, acetonitrile and dimethylformamide (mixing ratio of, for example, 1:1:3), and a mixed solvent of chloroform and water (mixing ratio of, for example, 20:1).
Although varying according to the types of solvent and the like, the reaction temperature is, normally, -20 to 100°C, preferably 0 to 50°C. Although varying according to the reaction temperature and the like, the reaction time is normally 15 minutes to 24 hours and preferably 30 minutes to 15 hours.
The subsequent deprotection reaction for removing PG', when PG' is, for example, a benzyl-based protecting group (preferably 2,4-dimethoxybenzyl or 4-methoxybenzyl), can be carried out by the same reaction as the one for removing a benzyl-based protecting group which is described in step 2-b.

Since a compound of formula (I) as claimed in the present invention or pharmaceutically acceptable salt thereof has superior PI3K inhibitory action, and particularly superior inhibitory action against the p110α of class Ia of PI3K, it is useful as a preventive agent or therapeutic agent of a proliferative disease, and is particularly useful as a preventive agent or therapeutic agent of cancer among the proliferative disease as a result of using a compound of the present invention alone or using concomitantly with various types of anticancer agents.

In the present specification, the "proliferative disease" refers to a disorder caused by deficiencies in the intracellular signal transduction system or the signal transduction mechanism of a certain protein. The proliferative disease includes, for example, cancers, psoriasis, restenosis, autoimmune diseases, and atherosclerosis. Examples of cancers include solid cancers, while examples of solid cancers include colon cancer, prostate cancer and non-small cell lung cancer.

In addition, a compound of formula (I) of the present invention is also useful as a preventive agent or therapeutic agent (particularly a therapeutic agent) of psoriasis, restenosis, autoimmune diseases and atherosclerosis, as well as diseases such as heart failure sequela, xenograft rejections, osteoarthritis, rheumatoid arthritis, respiratory diseases such as asthma, cystic fibrosis, hepatoma, cardiomegaly, Alzheimer's disease, diabetes, septic shock, HIV infection, inflammations caused by allergies and heart disease.

In particular, a compound of formula (I) of the present invention is useful as a preventive agent or therapeutic agent (particularly a therapeutic agent) of cancers in which PI3K, and particularly the p110α in class Ia of PI3K, is highly expressed.

Moreover, the present invention also relates to methods for preventing or treating the proliferative diseases described above, for example, cancer. Another aspect of the present invention includes methods for preventing or treating solid or hematopoietic PI3K-related cancers.

These methods include a step in which a pharmaceutical composition comprising as an active ingredient the compound of formula (I) or a pharmaceutically acceptable salt thereof, is administered to a patient requiring such treatment or a patient suffering from such a disease or condition.

A pharmaceutical composition of the present invention can be formulated and administered orally or parenterally (such as intravenously, intramuscularly, subcutaneously, rectally, nasally, intracisternally, vaginally, abdominally, intracystically or locally). Examples of preparations for oral administration include tablets, capsules, granules, powders, pills, aqueous or non-aqueous oral solutions and suspensions. Examples of preparations for parenteral administration include injections, ointments, gels, creams, suppositories, oral or nasal sprays, emulsions, oily agents and suspensions, as well as parenteral solutions filled into containers suitable for administration in individual small doses. In addition, the administration form can be adapted to various administration methods including controlled-release formulations in the manner of subcutaneous transplants.

The aforementioned preparations can be synthesized according to well-known methods using additives ordinarily used in pharmaceutical preparations, examples of which include vehicles, lubricants (coating agents), binders, disintegration agents, stabilizers, correctives, diluents, surfactants and emulsifiers.

Examples of such vehicles include starches such as starch, potato starch and cornstarch, lactose, crystalline cellulose and calcium hydrogen phosphate.

Examples of such coating agents include ethyl cellulose, hydroxypropyl cellulose, hydroxypropyl methyl cellulose, shellac, talc, Carnauba wax and paraffin.

Examples of such binders include polyvinyl pyrrolidone, Macrogol and the same compounds as listed for the aforementioned vehicles.

Examples of such disintegration agents include the same compounds as those listed for the aforementioned vehicles and chemically-modified starches and celluloses such as cross carmellose sodium, sodium carboxymethyl starch or crosslinked polyvinyl pyrrolidone.

Examples of such stabilizers include paraoxybenzoic acid esters such as methyl paraben or propyl paraben; alcohols such as chlorobutanol, benzyl alcohol or phenylethyl alcohol; benzalkonium chloride; phenols such as phenol, cresol, or chlorocresol; thimerosal; dehydroacetic acid; and sorbic acid.

Examples of such correctives include ordinarily used sweeteners, sour flavorings and fragrances.

Examples of such surfactants and emulsifiers include Polysorbate 80, Polyoxyl 40 Stearate and Lauromacrogol.

In addition, examples of solvents able to be used for producing liquid preparations include ethanol, phenol, purified water and distilled water.

In the case of using a pharmaceutical composition of the present invention as a PI3K inhibitor or therapeutic agent or preventive agent of a proliferative diseases such as cancer, the amount of compound of formula (I) of the present invention, or pharmaceutically acceptable salt thereof, can be suitably altered according to symptoms, age, body weight, relative state of health, presence of other drugs, administration method and the like. For example, the typical effective amount for a patient (warm-blooded animal and particularly a human) of a compound of formula (I) in the case of an oral preparation is preferably 0.01 to 500 mg, and more preferably 0.05 to 50 mg, per kg of body weight per day. In the case of parenteral administration, the typical effective amount is preferably 0.01 to 500 mg and more preferably 0.05 to 50 mg per kg of body weight per day. This amount is preferably administered once a day or divided into several administrations according to symptoms.

The pharmaceutical composition of the present invention can be used concomitantly with other radiotherapy, chemotherapy, vascularization inhibitors and anticancer agents.

### EXAMPLES

Hereinbelow, the present invention is described in more detail by Examples, but the present invention is not limited to these Examples. In the present specification, "N" means "normality", and "M" means "mol/L".
Further, NMR analysis was carried out using JNM-EX270 (270 MHz), JNM-GSX400 (400 MHz) from JEOL, Ltd. or NMR (400 MHz) from Bruker company, and NMR data is represented by ppm (parts per million). A deuterated lock signal from a sample solvent was referred to, with tetramethyl silane being set as an internal standard substance (0 ppm).

Mass spectrum data was obtained using JMS-DX303, JMS-SX/SX102A from JEOL Ltd. or Quttromicro from Micromass Ltd., and mass spectrum data provided with high performance liquid chromatography was obtained using a micromass (ZMD from Micromass Ltd.) equipped with 996-600E gradient high performance liquid chromatography from Waters Corporation or a micromass (ZQ from Micromass Ltd.) equipped with 2525 gradient high performance liquid chromatography from Waters Corporation.

For the condition for high performance liquid chromatography (LC-MS), the following condition was used.

### Condition for high performance liquid chromatography

Column: Combi ODS (ODS, 5 µm, 4.6 mm I.D. x50 mm, from Wako Pure Chemicals Industries, Ltd.), COSMOSIL (ODS, 5 µm, 4.6 mmI.D. x50 mm, from Nacalai Tesque, Inc.), Inertsil C18 (ODS, 5 µm, 4.6 mm I.D.x50 mm, from GL SCIENCES INC.), or SunFire C18 (ODS, 5 µm, 4.6 mm I.D.x50 mm, from Waters Corporation)
Mobile phase: a water containing 0.05% trifluoroacetic acid (A) and acetonitrile containing 0.05% trifluoroacetic acid (B)
Elution method: stepwise solvent gradient elution from 10% of B to 95% of B (3.5 min.), from 95% of B to 10% ofB (1 min.), kept at 10%ofB (0.5 min.)
Flow rate: 4.0 mL/min.

In preparation of a compound, a functional group was protected by a protective group as necessary, and a protected form of a target molecule was prepared, followed by removal of the protective group. Selection and desorption operation of a protective group were carried out according to the method described, for example, in Greene and Wuts, "Protective Groups in Organic Synthesis", 3rd edition, John Wiley & Sons, 1999.

### Example 1

### Synthesis of 5-{7-[2-(4-Ethyl-piperazin-1-yl)-pyridin-4-yl]-2-morpholin-4-yl-7H-pyrrolo[2,3-d]pyrimidin-4-yl}-pyrimidin-2-ylamine (1-1)

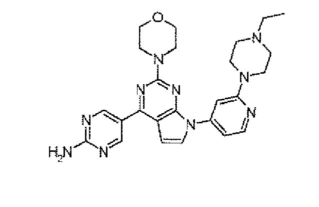

### Step A

### 5-(2-Hydroxy-ethyl)-2-morpholin-4-yl-pyrimidin-4,6-diol

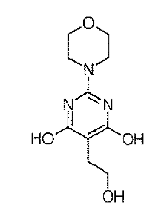

A condenser and a rubber septum were connected to a 300 ml two-neck flask, into which a magnetic bar for stirring was put. The inside of the system was replaced with nitrogen gas, and then morpholin-4-carboxamidine hydrobromide (42.2 g, 0.20 mol) and MeOH (160 ml) were added. After stirring at room temperature for 10 minutes to dissolve the mixture, 28% NaOMe-MeOH (120 ml, 0.62 mol) was added at room temperature. With stirring under a nitrogen atmosphere, was added 2-oxotetrahydrofuran-3-carboxylic acid methyl ester (Journal of Organic Chemistry (1978), 43(2), 346)(34.8 g, 0.24 mol), which was washed using MeOH (10 ml). This mixture was heated to reflux for three hours using an oil bath. After cooled to room temperature, water (300 ml) was added to the reaction mixture, and stirred at room temperature for 30 minutes. To this deep orange-red solution, acetic acid (24 ml, 0.42 mol) was added at room temperature, and stirred for 2 hours. The precipitated solid was filtered, and washed using water (30 ml). The obtained solid was dried under reduced pressure, to obtain 5-(2-hydroxy-ethyl)-2-morpholin-4-yl-pyrimidin-4,6-diol (35.2 g, 0.146 mol, 73%) as a white powder.
¹H-NMR (DMSO-d6) δ (ppm): 2.39 (2H, t, J=7.63Hz), 3.33 (2H, t, J=7.63Hz), 3.51 (4H, t, J=4.70Hz), 3.61 (4H, t, J=4.70Hz), 10.54 (2H, bs).
ESI (LC-MS positive mode) m/z : 242 [(M+H)⁺].

### Step B

### 4-[4,6-Dichloro-5-(2-chloroethyl)-pyrimidin-2-yl]-morpholine

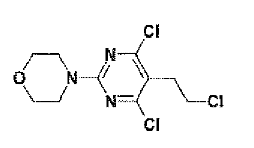

A temperature sensor and a rubber septum were connected to 3 L three-neck flask, and 5-(2-hydroxy-ethyl)-2-morpholin-4-yl-pyrimidin-4,6-diol (50 g, 0.207 mol) prepared in Step A, toluene (250 ml) and DIPEA (53.2 ml, 0.311 mol) were added. This was cooled to 0°C with an ice bath, and POCl₃(77.3 ml, 0.829 mmol) was slowly added dropwise under stirring with a mechanical stirrer under a nitrogen stream such that the internal temperature was not more than 30°C. The ice bath was removed to raise the temperature to room temperature, and then to 100°C. After stirring for 7 hours, the stirring was continued overnight at room temperature. After further stirring at 100°C for 4 hours, it was cooled to 0°C in an ice bath. To this, water (750 ml) was slowly added dropwise such that the internal temperature was not more than 30°C. After stirring for 1 hour, an organic layer was extracted with toluene (750 ml). The organic layer was washed with brine (750 ml) followed by concentration, and further azeotropy was carried out twice using 1 L toluene, to obtain 4-[4,6-dichloro-5-(2-chloroethyl)-pyrimidin-2-yl]-morpholine as a pale brown powder (55.5 g, 91%). ¹H-NMR (CDCl₃) δ: 3.20 (2H, t, J = 7.9 Hz), 3.66 (2H, t, J = 7.9Hz), 3.70-3.81 (8H, m).
ESI (LC-MS positive mode) m/z 296 [(M+H)⁺].

### Step C

### [6-Chloro-5-(2-chloroethyl)-2-morpholin-4-yl]-pyrimidin-4-yl-(4-methoxybenzyl)-amine

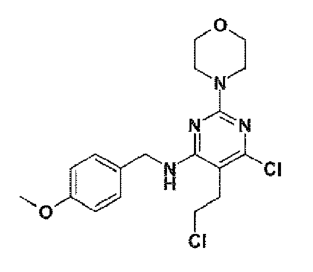

4-[4,6-Dichloro-5-(2-chloroethyl)-pyrimidin-2-yl]-morpholine (2.9 g) prepared in Step B, 4-methoxybenzylamine (1.91 ml) and diisopropylethylamine (3.40 ml) were dissolved in acetonitrile (40 ml), and refluxed for 10 hours. Further, 4-methoxybenzylamine (0.64 ml) and diisopropylethylamine (0.85 ml) were added, and subsequently refluxed for 1 hour. After the solvent was concentrated under reduced pressure, the residue was dissolved in ethyl acetate (150 ml), washed with saturated aqueous ammonium chloride solution (200 ml) and brine (200 ml), and dried with sodium sulfate. The residue which was obtained by filtering off of the drying agent and concentration was purified by silica gel column chromatography (dichloromethane/methanol=100/0 to 100/1), to obtain the desired compound as a yellow solid (2.13 g, 55%).
¹H-NMR (270MHz), CDCl3) δ (ppm): 7.23 (2H, d, J=8.7Hz), 6.87 (2H, d, J=8.7Hz), 5.16 (1H, t, J=5.4Hz), 4.55 (2H, d, J=5.4Hz), 3.80 (3H, s), 3.68-3.78 (8H, m), 3.62 (2H, t, J=7.3Hz), 2.91 (2H, t, J=7.3Hz).
ESI (LC-MS positive mode) m/z 361 [(M+H)⁺].

### Step D

### 4-Chloro-7-(4-methoxybenzyl)-2-morpholin-4-yl-6,7-dihydro-5H-pyrrolo[2,3-d]pyrimidine

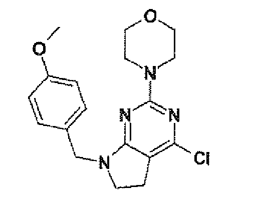

[6-Chloro-5-(2-chloroethyl)-2-morpholin-4-yl]-pyrimidin-4-yl-(4-methoxybenzyl)-amine prepared in Step C was dissolved in acetonitrile (290 ml), and cesium carbonate (5.65 g) and sodium iodide (1.83 g) were added, and refluxed for 10 hours. The reaction mixture was diluted with water (200 ml), and extracted with ethyl acetate (200 mlx2). After the organic layer was washed with brine, it was dried with sodium sulfate. After the drying agent was filtered off, the filtrate was concentrated under reduced pressure to obtain a pale yellow powder (2.10 g). The crude product was used for the next reaction without purification.
¹H-NMR (270MHz, CDCl₃) δ (ppm): 7.19 (2H, d, J=8.5Hz), 6.86 (2H, d, J=8.5Hz), 4.48 (2H, s), 3.80 (3H, s), 3.70-3.80 (8H, m), 3.43 (2H, t, J=8.4Hz), 2.87 (2H, t, J=8.4Hz).
ESI (LC-MS positive mode) m/z 361 [(M+H)⁺],

### Step E

### 4-Chloro-7-2-morpholin-4-yl-6,7-dihydro-5H-pyrrolo[2,3-d]pyrimidine

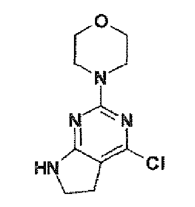

4-Chloro-7-(4-methoxybenzyl)-2-morpholin-4-yl-6,7-dihydro-5H-pyrrolo[2,3-d]pyrimidine (1.87 g) prepared in Step D was dissolved in trifluoroacetic acid (5.2 ml), and concentrated sulfuric acid (290 µl, 1.05 equivalents) was added, followed by refluxing for 3 hours. Excess amount of solvent was concentrated under reduced pressure, and the resulting residue was poured onto ice water (ca. 25 ml), followed by neutralization with 5M sodium hydroxide with ice cooling. The reaction mixture was extracted twice with ethyl acetate/tetrahydrofuran (4/1150 ml), and the organic layer was washed with brine, followed by drying over sodium sulfate. After the drying agent was filtered off, the filtrate was concentrated under reduced pressure to obtain a pale brown powder (1.78 g). The crude product was used for the next reaction without purification.
¹H-NMR (270MHz, CDC13) δ (ppm): 4.91 (1H, brs), 3.70 (8H, s), 3.64 (2H, t, J=8.4Hz), 2.99 (2H, t, J=8.4Hz).
ESI (LC-MS positive mode) m/z 241 [(M+H)⁺].

### Step F

### 1-(4-Chloro-2-morpholin-4-yl-5,6-dihydro-pyrrolo[2,3-d]pyrimidin-7-yl)-ethanone

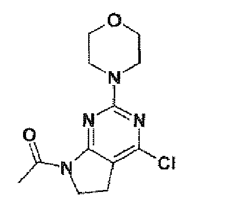

4-Chloro-2-morpholin-4-yl-6,7-dihydro-5H-pyrrolo[2,3-d]pyrimidine (2.94 g) prepared in Step E, dimethylaminopyridine (28 mg) and pyridine (2.48 ml) were added to acetonitrile (50 ml), and acetyl chloride (1.67 ml) was added dropwise slowly with ice cooling. The reaction mixture was raised to room temperature, followed by stirring for 30 minutes. After the reaction mixture was diluted with water (200 ml) and ethyl acetate (200 ml), insolubles were filtered off through Celite pad, and the Celite pad was washed with ethyl acetate. The organic layer of the filtrate was separated, and the aqueous layer was extracted with ethyl acetate (200 ml). The organic layers were combined, and washed with brine, followed by drying over sodium sulfate. After the drying agent was filtered off, the filtrate was concentrated under reduced pressure, and the resulting residue was purified by silica gel column chromatography (ethyl acetate/hexane=3/0 to 2/1), to obtain the desired compound (1.67g) as a pale yellow powder.
¹H-NMR (270MHz, CDCl₃) δ (ppm): 4.04 (2H, t, J=8.5Hz), 3.66-3.78 (8H, brs), 2.92 (2H, t, J=8.5Hz), 2.62 (3H, s).
ESI (LC-MS positive mode) m/z 283 [(M+H)⁺].

### Step G

### 1-(4-{2-[Bis-(4-methoxy-benzyl)-amino]-pyrimidin-5-yl}-2-morpholin-4-yl-5,6-dihydro-pyrrolo[2,3-d]pyrimidin-7-yl)-ethanone

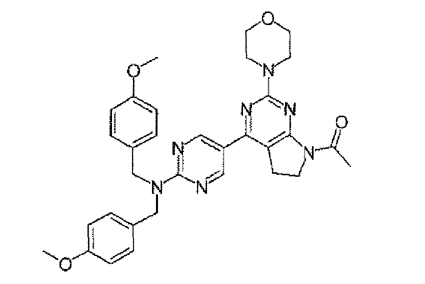

To 1-(4-chloro-2-morpholin-4-yl-5,6-dihydro-pyrrolo[2,3-d]pyrimidin-7-yl)-ethanone (300 mg, 1.06 mmol, 1.0 equivalent) prepared in Step F, bis-(4-methoxybenzyl)-[5-(4,4,5,5-tetramethyl-[1,3,2]dioxaborolan-2-yl)-pyrimidin-2-yl]amine (538 mg, 1.17 mmol, 1.1 equivalents), palladium acetate (2.4 mg, 0.0106 mmol, 1 mol%), S-Phos (8.7 mg, 0.0212 mmol, 2 mol%) and potassium phosphate (450 mg, 2.12 mmol, 2.0 equivalents), dimethyl formamide (5 ml) was added, and subjected to degassing under ultrasonic irradiation. This was stirred at 100°C for 1.5 hours, followed by addition of water, to filter the solid, which was dissolved in dichloromethane, and dried over anhydrous sodium sulfate. Concentration was carried out under reduced pressure, followed by purification by column chromatography (dichloromethane/methanol=50/1), to obtain the desired compound as a colorless solid (560 mg, yield 91%).
¹H-NMR (CDCl₃) δ (ppm): 8.98 (2H, s), 7.19 (4H, d, J=8.8Hz), 6.85 (4H, d, J=8.8Hz), 4.84 (4H, s), 4.10 (2H, t, J=8.5Hz), 3.84-3.76 (8.0H, m), 3.80 (6H, s), 3.18 (2H, t, J=8.5Hz), 2.69 (3.0H, s).
ESI (LC-MS positive mode) m/z 582 [(M+H)⁺].

### Step H

### Bis-(4-methoxy-benzyl)-[5-(2-morpholin-4-yl-6,7-dihydro-5H-pyrrolo[2,3-d]pyrimidin-4-yl)-pyrimidin-2-yl]-amine

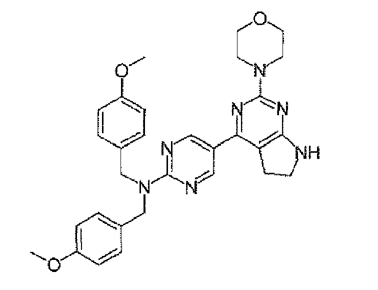

To a solution of 1-(4-{2-[bis-(4-methoxy-benzyl)-aminol-pyrimidin-5-yl}-2-morpholin-4-yl-5,6-dihydro-pyrrolo[2,3-d]pyrimidin-7-yl)-ethanone (335 mg, 0.576 mmol)) prepared in Step G in tetrahydrofuran (12 ml), 5M aqueous sodium hydroxide solution (6 ml) was added, followed by refluxing overnight. To this, IN hydrochloric acid was added for neutralization, and the obtained solid was filtered, which was washed with acetonitrile, to obtain the desired compound as a colorless solid (290 mg, yield 93%).
¹H-NMR (CDCl₃) δ (ppm): 8.97 (2H, s), 7.18 (4H, d, J=8.3Hz), 6.85 (4H, d, J=8.3Hz), 4.83 (4H, s), 3.79 (6H, s), 3.79-3.73 (8H, m), 3.68 (2H, t, J=8.3Hz), 3.24 (2H, t, J=8.3Hz).
ESI (LC-MS positive mode) m/z 540 [(M+H)⁺].

### Step I

### {5-[7-(2-Chloro-pyridin-4-yl)-2-morpholin-4-yl-6,7-dihydro-5H-pyrrolo[2,3-d]pyrimidin-4-yl]-pyrimidin-2-yl}-bis-(4-methoxy-benzyl)-amine

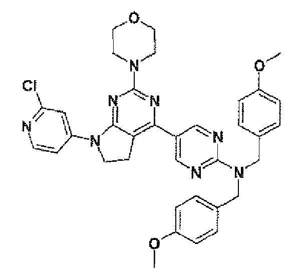

Bis-(4-methoxybenzyl)-[5-(2-morpholin-4-yl-6,7-dihydro-5H-pyrrolo [2,3-d]pyrimidin-4-yl)-pyrimidin-2-yl]-amine (1.65 g) prepared in Step H was suspended in dimethylformamide (20 ml), followed by addition of 2-chloro-4-iodopyridine (805 mg), palladium acetate (35 mg), triphenylphosphine (81 mg) and potassium phosphate (1.95 g), and argon gas was blown for 10 minutes while irradiating ultrasonic wave. The reaction mixture was stirred at 100°C for 1 hour, and cooled to room temperature, followed by addition of water (50 ml). The mixture was extracted with ethyl acetate (100 ml) and dichloromethane (100 ml), and the combined organic layers were washed with brine, followed by drying over sodium sulfate. After filtering off the drying agent, the filtrate was concentrated under reduced pressure, and the resulting residue was purified by silica gel column chromatography (dichloromethane/methanol=50/1), followed by suspension of the residue in ethyl acetate/hexane (10 ml/50 ml). The precipitate was filtered, and washed with hexane, followed by drying under reduced pressure, to obtain a yellow powder (1.75g, 88%). ¹H-NMR (300MHz, CDC13) δ (ppm): 8.99 (2H, s), 8.27 (1H, d, J=5.7Hz), 7.82 (1H, d, J=1.9Hz), 7.71 (1H, dd, J=5.7, 1.9Hz), 7.20 (4H, d, J=8.4Hz), 6.86 (4H, d, J=8.4Hz), 4.84 (4H, s), 4.08 (2H, t, J=8.4Hz), 3.81-3.89 (8H, m), 3.80 (6H, s), 3.36 (2H, t, J=8.4Hz).
EST (LC-MS positive mode) m/z 651 [(M+H)⁺].

### Step J

### (5-{7-[2-(4-Ethyl-piperazin-1-yl)-pyridin-4-yl]-2-morpholin-4-yl-6,7-dihydro-5H-pyrrolo[2,3-d]pyrimidin-4-yl}-pyrimidin-2-yl)-bis-(4-methoxy-benzyl)-amine

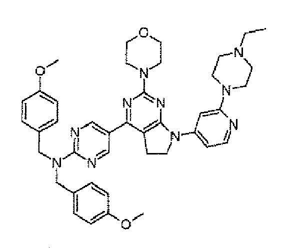

To a solution of {5-[7-(2-chloro-pyridin-4-yl)-2-morpholin-4-yl-6,7-dihydro-5H-pyrrolo[2,3-d]pyrimidin-4-yl]-pyrimidin-2-yl}-bis-(4-methoxy-benzyl)-amine (200 mg) prepared in Step I, sodium t-butoxide (59 mg) and palladium dibenzylidene acetone complex (16 mg) suspended in toluene (4 ml), argon gas was blown for 5 minutes. 1-Ethyl piperazine (59 µl) and 2,8,9-triisobutyl-2,5,8,9-tetraaza-1-phospha-bicyclo[3.3.3]undecane (26 mg) were added, followed by stirring at 100°C for 5 hours. The reaction mixture was cooled to room temperature, and water was added, followed by extraction with dichloromethane. The combined organic layers were washed with brine, and dried over magnesium sulfate. After the drying agent was filtered off, the filtrate was concentrated under reduced pressure, and the resulting residue was purified by silica gel column chromatography (dichloromethane/methanol=100/1), to obtain a yellow solid (211 mg, 94%).
¹H-NMR (300MHz, CDCl3) δ (ppm): 8.99 (2H, s), 8.13 (1H, d, J=5.7Hz), 7.47 (1H, bs), 7.28 (1H, bs), 7.20 (4H, m), 6.85 (4H, m), 4.84 (4H, s), 4.09 (2H, m), 3.87 (4H, m), 3.80 (10H, s), 3.59 (4H, m), 3.31 (2H, m), 2.47 (4H, m), 2.48 (2H, q, J=7.25Hz), 1.12 (3H, t, J=7.25Hz).
ESI (LC-MS positive mode) m/z 730 [(M+H)⁺].

### Step K

### (5- {7-[2-(4-Ethyl-piperazin-1-yl)-pyridin-4-yl]-2-morpholin-4-yl-6,7-dihydro-5H-pyrrolo[2,3-d]pyrimidin-4-yl}-pyrimidin-2-yl)-(4-methoxy-benzyl)-amine

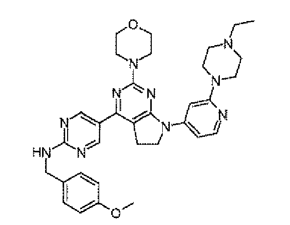

(5- {7-[2-(4-Ethyl-piperazin-1-yl)-pyridin-4-yl]-2-morpholin-4-yl-6,7-dihydro-5H-pyrrolo[2,3-d]pyrimidin-4-yl}-pyrimidin-2-yl)-bis-(4-methoxy-benzyl)-amine (211 mg) prepared in Step J was dissolved in trifluoroacetic acid (1 ml), and stirred at 50°C for 5 hours. Water was added, followed by extraction with dichloromethane, and the organic layer was washed with saturated aqueous sodium hydrogencarbonate solution and brine, and dried over magnesium sulfate. The drying agent was filtered off, and the filtrate was concentrated under reduced pressure, and the resulting residue was purified by silica gel column chromatography (dichloromethane/methanol=10/1), to obtain (5-{7-[2-(4-ethyl-piperazm-1-yl)-pyridin-4-yl]-2-morpholin-4-yl-6,7-dihydro-5H-pyrrolo[2,3-d]pyrimidin-4-yl}-pyrimidin-2-yl)-(4-methoxy-benzyl)-amine (197 mg) as a pale yellow solid.
¹H-NMR (CDCl₃) δ (ppm): 8.88 (2H, s), 8.05 (1H, d, J=5.7Hz), 7.73 (1H, s), 7.30 (2H, m), 7.19 (1H, s), 6.99 (2H, m), 4.63 (2H, s), 4.17 (2H, m), 3.85 (11H, m), 3.40 (8H, m), 3.19 (2H, q, J=7.25Hz), 1.41 (3H, t, J=7.25Hz).

### Step L

### (5-{7-[2-(4-Ethyl-piperazin-1-yl)-pyridin-4-yl]-2-morpholin-4-yl-7H-pyrrolo[2,3-d]pyrimidin-4-yl} -pyrimidin-2-ylamine

(5- {7-[2-(4-Ethyl-piperazin-1-yl)-pyridin-4-yl]-2-morpholin-4-yl-6,7-dihydro-5H-pyrrolo[2,3-d]pyrimidin-4-yl}-pyrimidin-2-yl)-(4-methoxy-benzyl)-amine (30 mg) prepared in Step K was dissolved in chloroform/water=20/1 (2.1 ml), and DDQ (33 mg) was added, followed by stirring at room temperature for 4 hours. 1N-NaOH was added, followed by extraction with chloroform, and the organic layer was dried over magnesium sulfate. After the drying agent was filtered off, the filtrate was concentrated under reduced pressure, and the resulting residue was purified by silica gel column chromatography (dichloromethane/methanol=30/1), to obtain (5-{7-[2-(4-ethyl-piperazin-1-yl)-pyridin-4-yl]-2-morpholin-4-yl-7H-pyrrolo[2,3-d]pyrimidin-4-yl}-pyrimidin-2-ylamine (9 mg, 38%) as a pale yellowish white solid. ¹H-NMR (CDCl₃) δ (ppm) : 9.05 (s, 2H), 8.27 (d, 1H, J=5.7Hz), 7.46 (d, 1H, J=1.1Hz), 7.33 (d, 1H, J=3.8Hz), 7.02 (dd, 1H, J=5.7Hz, J=1.9Hz), 6.72 (d, 1H, J=3.8Hz), 5.37 (s, 2H), 3.89 (m, 8H), 3.69 (t, 4H, J=5.3Hz), 2.62 (t, 4H, J=4.5Hz), 2.52 (q, 2H, J=7.2Hz), 1.17 (t, 3H, J=7.2Hz)
ESI (LC-MS positive mode) m/z : 487 [(M+H)⁺].

### Example 2

### Synthesis of {3-[4-(2-amino-pyrimidin-5-yl)-2-morpholin-4-yl-pyrrolo[2,3-d]pyrimidin-7-yl]-4-methyl-phenyl}-morpholin-4-yl-methanone (2-1)

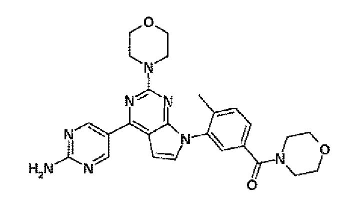

### Step A

### (3-Bromo-4-methyl-phenyl)-morpholin-4-yl-methanone

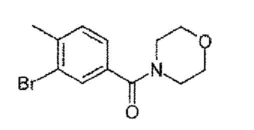

To a dichloromethane solution (80 ml) of 3-bromo-4-methylbenzoic acid (2.8 g, 13.0 mmol), WSCI (3.5 g, 18.2 mmol), morpholine (1.36 ml, 15.6 mmol) and N,N-dimethylaminopyridine (794 mg, 6.5 mmol) were added, followed by stirrig at room temperature for 1 hour. To the reaction mixture, saturated aqueous ammonium chloride solution was added, followed by extraction twice with ethyl acetate (100 ml). The organic layer was washed with brine (100 ml), and dried over sodium sulfate. After the drying agent was filtered off, the filtrate was concentrated under reduced pressure, and the resulting residue was purified by silica gel column chromatography (dichloromethane/methanol=50/1), to obtain (3-bromo-4-methyl-phenyl)-morpholin-4-yl-methanone as a yellow brown solid (3.2 mg, 86%).
¹H-NMR (CDCl3) δ (ppm): 7.59 (1H, d, J=1.5Hz), 7.26 (1H, s), 7.25 (1H, d, J=1.5Hz), 3.69 (8H, s), 2.42 (3H, s).
ESI (LC-MS positive mode) m/z 284, 286 (M)⁺.

### Step B

### {3-[4-(2-Amino-pyrimidin-5-yl)-2-morpholin-4-yl-pyrrolo[2,3-d]pyrimidin-7-yl]-4-methyl-phenyl}-morpholin-4-yl-methanone

A dimethylformamide (50 ml) solution of bis-(4-methoxy-benzyl)-[5-(2-morpholin-4-yl-6,7-dihydro-5H-pyrrolo [2,3-d]pyrimidin-4-yl)-pyrimidin-2-yl]-amine (3.0 g, 5.56 mmol) prepared according to Example 1, Step H, (3-bromo-4-methylphenyl)-morpholin-4-yl-methanone (2.4 g, 8.34 mmol) obtained in Step A, Pd(OAc)₂ (125 mg, 0.556 mmol), X-Phos (529 mg, 1.11 mmol) and potassium phosphate (2.4 g, 11.12 mmol) was degassed under ultrasonic irradiation, followed by stirring at 100°C for 14 hours. The reaction mixture was cooled to room temperature, and subsequently saturated aqueous ammonium chloride solution was added, followed by extraction twice with ethyl acetate (200 ml). The organic layer was washed with brine (200 ml), and dried over sodium sulfate. After the drying agent was filtered off, the filtrate was concentrated under reduced pressure, and the resulting residue was purified by silica gel column chromatography (dichloromethane/methanol=100/1 to 30/1), to obtain [3-(4-{2-[bis-(4-methoxy-benzyl)-amino]-pyrimidin-5-yl}-2-morpholin-4-yl-5,6-dihydro-pyrrolo[2,3-d]pyrimidin-7-yl)-4-methyl-phenyl]-morpholin-4-yl-methanone as a brown oil (5.2 g, 100%).
This was dissolved in TFA (25 ml), and refluxed for 14 hours in the presence of N-acetylcysteine (1.8 g, 11.1 mmol). The reaction mixture was concentrated under reduced pressure, and the resulting residue was purified by silica gel column chromatography (dichloromethane/methanol=5/1), to obtain the desired compound as a yellow powder (124 mg, 4%).
¹H-NMR (CDCl3) δ (ppm): 9.08 (2H, s), 7.43 (2H, d, J = 1.2 Hz), 7.37 (1H, s), 7.03 (1H, d, J = 3.8 Hz), 6.71 (1H, d, J = 3.8 Hz), 5.30 (2H, s), 3.76 (16H, br s), 2.22 (3H, s).
ESI (LC-MS positive mode) m/z 501(M+H)⁺.
Further, in the above reaction, other than the desired compound, there was also obtained {3-[4-(2-amino-pyrimidin-5-yl)-2-morpholin-4-yl-5,6-dihydropyrrolo[2,3-d]pyrimidin-7-yl]-4-methyl-phenyl}-morpholin-4-yl-methanone as a product (ESI (LC-MS positive mode) m/z 503(M+H)⁺). The obtained {3-[4-(2-amino-pyrimidin-5-yl)-2-morpholin-4-yl-5,6-dihydro-pyrrolo[2,3-d]pyrimidin-7-yl]-4-methyl-phenyl}-morpholin-4-yl-methanone may be reacted with 1 to 3 equivalents of DDQ in a mixed solvent of dichloromethane and dimethylformamide (e.g., mixture ratio of 9 : 1 to 1 : 1; acetonitrile may further be mixed), to synthesize {3-[4-(2-amino-pyrimidin-5-yl)-2-morpholin-4-yl-pyrrolo[2,3-d]pyrimidin-7-yl]-4-methylphenyl}-morpholin-4-yl-methanone which is the desired compound.

### Example 3

### Synthesis of 5- {7-[4-(1,1-dioxo-1λ⁶-thiomorpholin-4-ylmethyl)-phenyl]-2-morpholin-4-yl-7H-pyrrolo[2,3-d]pyrimidin-4-yl}-pyrimidin-2-ylamine (3-1)

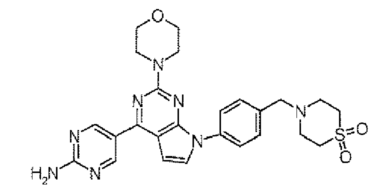

### Step A

### 4-(4-{2-[Bis-(4-methoxy-benzyl)-amino]-pyrimidin-5-yl}-2-morpholin-4-yl-5,6-dihydro-pyrrolo[2,3-d]pyrimidin-7-yl)-benzaldehyde

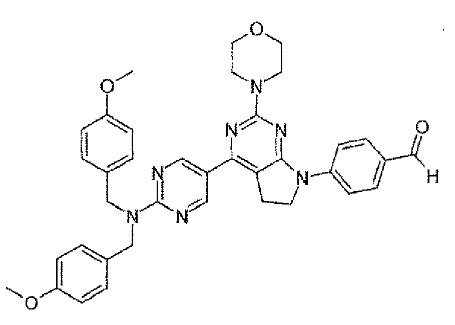

A dimethylformamide (1 ml) solution of bis-(4-methoxy-benzyl)-[5-(2-morpholin-4-yl-6,7-dihydro-6H-pyrrolo[2,3-d]pyrimidin-4-yl)-pyrimidin-2-yl]-amine (50 mg, 0.093 mmol) prepared according to Example 1, Step H, 4-bromobenzaldehyde (22 mg, 0.121 mmol), Pd₂dba₃ (8.5 mg, 0.0093 mmol), S-Phos (7.6 mg, 0.0186 mmol) and potassium phosphate (39 mg, 0.186 mmol) was degassed under ultrasonic irradiation, followed by stirring at 100°C for 16 hours. The reaction mixture was cooled to room temperature, and subsequently saturated aqueous ammonium chloride solution was added, followed by extraction twice with ethyl acetate (10 ml). The organic layer was washed with brine (10 ml), and dried over sodium sulfate, followed by distilling off under reduced pressure. The resulting residue was washed with ether, to obtain the desired compound as a dark brown powder (29 mg, 49%).
¹H-NMR (DMSO-D6) δ (ppm): 9.87 (1H, s), 8.99 (2H, s), 8.06 (2H, d, J=8.8Hz), 7.91 (2H, d, J =8.8Hz), 7.21 (4H, d, J=8.3Hz), 6.89 (4H, d, J=8.3Hz), 4.79 (4H, s), 3.73 (6H, s), 3.34 (4H, s).
ESI (LC-MS positive mode) m/z 644 (M+H)⁺.

### Step B

### (5- {7-[4-(1,1-Dioxo-1λ⁶-thiomorpholin-4-ylmethyl)-phenyl]-2-morpholin-4-yl-6,7-dihydro-5H-pyrrolo[2,3-d]pyrimidin-4-yl}-pyrimidin-2-yl)-bis-(4-methoxy-benzyl)-amine

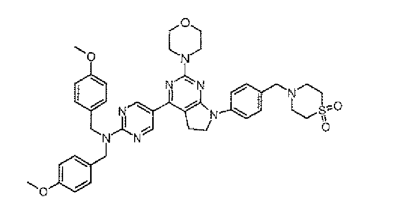

To a dichloromethane solution (80 ml) of 4-(4-{2-[bis-{4-methoxy-benzyl)-amino]-pyrimidin-5-yl}-2-morpholin-4-yl-5,6-dihydro-pyrrolo[2,3-d]pyrimidin-7-yl)-benzaldehyde (0.80 g, 1.24 mmol) prepared in Step A, thiomorpholin-1,1-dioxide (0.34 g, 2.49 mmol), sodium triacetoxyborohydride (0.55 g, 2.49 mmol) and aetic acid (0.14 ml, 2.49 mmol) were added, and heated to reflux for 24 hours. After the reaction system was cooled, the precipitated solid was filtered, and washed with dichloromethane (20 ml). The filtrate and washings were combined, which was washed sequentially with saturated aqueous ammonium chloride solution (50 mL) and saturated aqueous sodium chloride solution (50 mL), and subsequently the organic layer was dried over magnesium sulfate. After filtering off the drying agent, the filtrate was concentrated under reduced pressure, and the resulting residue was purified by amino silica gel column chromatography (dichloromethane/n-hexane=4/1), to obtain [(5-{7-[4-(1,1-dioxo-1λ⁶-thiomorpholin-4-ylmethyl)-phenyl]-2-morpholin-4-yl-6,7-dihydro-5H-pyrrolo[2,3-d]pyrimidin-4-yl}-pyrimidin-2-yl)-bis-(4-methoxybenzyl)-amine as a yellow solid (0.71 g, 75%).
¹H-NMR (CDCl3) δ: 9.00 (2H, s), 7.78 (2H, d, J = 8.6 Hz), 7.32 (2H, d, J = 8.6 Hz), 7.19 (4H, d, J = 8.6 Hz), 6.90-6.82 (4H, m), 4.84 (4H, s), 4.11 (2H, t, J = 8.2 Hz), 3.89-3.76 (14H, m), 3.64 (2H, s), 3.31 (2H, t, J = 8.2 Hz), 3.10-2.97 (8H, m).
ESI (LC-MS positive mode) m/z 763 (M+H)⁺.

### Step C

### (5-{7-[4-(1,1-Dioxo-1λ⁶-thiomorpholin-4-ylmethyl)-phenyl]-2-morpholin-4-yl-6,7-dihydro-5H-pyrrolo[2,3-d]pyrimidin-4-yl}-pyrimidin-2-yl)-amine

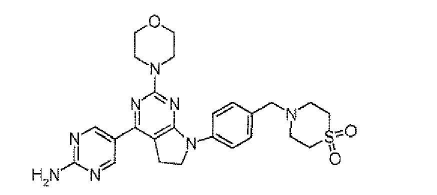

To an ethyl acetate solution (2 ml) of [(5-{7-[4-(1,1-dioxo-1λ⁶-thiomorpholin-4-ylmethyl)-phenyl]-2-morpholin-4-yl-6,7-dihydro-5H-pyrrolo[2,3-d]pyrimidin-4-yl}-pyrimidin-2-yl)-bis-(4-methoxy-benzyl)-amine (100.5 mg, 0.132 mmol)) prepared in Step B, concentrated sulfuric acid (0.50 ml, 9.35 mmol) was added, and stirred at 70°C for 3 hours. After cooling to room temperature, 5% aqueous potassium phosphate solution (30 ml) was added to the reaction mixture. The precipitated solid was filtered, and washed with water (5 ml). This was suspended in dichloromethane (2.5 ml), and the obtained solid was filtered, followed by washing with dichloromethane (3 ml), to obtain (5-{7-[4-(1,1-dioxo-1λ⁶-thiomorpholin-4-ylmethyl)-phenyl]-2-morpholin-4-yl-6,7-dihydro-5H-pyrrolo[2,3-d]pyrimidin-4-yl}-pyrimidin-2-yl)-amine as a pale yellow solid (48.5 mg, 70%).
¹H-NMR (DMSO-D6) δ: 8.81 (2H, s), 7.81 (2H, d, J = 8.6 Hz), 7.34 (2H, d, J = 8.6 Hz), 7.06 (2H, s), 4.08 (2H, t, J = 8.1 Hz), 3.70 (8H, br s), 3.63 (2H, s), 3.31-3.24 (2H, m), 3.14-3.07 (4H, m), 2.91-2.84 (4H, m).
ESI (LC-MS positive mode) m/z 523 (M+H)⁺.

### Step D

### 5- {7-[4-(1,1-Dioxo-1λ⁶-thiomorpholin-4-ylmethyl)-phenyl]-2-morpholin-4-yl-7H-pyrrolo[2,3-d]pyrimidin-4-yl}-pyrimidin-2-yl-amine

To dichloromethane/acetonitrile/dimethylformamide mixed solution (1:1 : 3, 5 ml) of(5-{7-[4-(1,1-dioxo-1λ⁶-thiomorpholin-4-ylmethyl)-phenyl]-2-morpholin-4-yl-6,7-dihydro-5H-pyrrolo[2,3-d]pyrimidin-4-yl}-pyrimidin-2-yl)-amine (8.6 mg, 0.017 mmol) prepared in Step C, DDQ (5.6 mg, 0.025 mmol) was added, and stirred at room temperature for 1 hour. The reaction mixture was concentrated under reduced pressure, and the resulting residue was purified by preparative thin layer chromatography (dichloromethane/methanol=9/1), to obtain 5-{7-[4-(1,1-dioxo-1λ⁶-thiomorpholin-4-ylmethyl)-phenyl]-2-morpholin-4-yl-7H-pyrrolo[2,3-d]pyrimidin-4-yl}-pyrimidin-2-yl-amine as a pale yellow solid (6.2 mg, 72%).
¹H-NMR (DMSO-D6) δ: 9.04 (2H, s), 7.88 (2H, d, J = 8.0 Hz), 7.67 (1H, d, J = 3.0 Hz), 7.50 (2H, d, J = 8.0 Hz), 7.23 (2H, br s), 6.96 (1H, d, J = 3.0 Hz), 3.75-3.71 (10H, br m), 3.18-3.10 (4H, m), 2.96-2.89 (4H, m).
ESI (LC-MS positive mode) m/z 521 (M+H)⁺.

### Example 4

### Synthesis of 5-(7-methanesulfonyl-2-morpholin-4-yl-7H-pyrrolo[2,3-d]pyrimidin-4-yl)-pyrimidin-2-ylamine(4- 1)

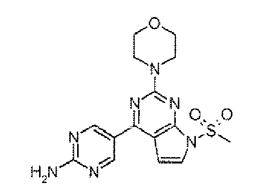

### Step A

### 4-Chloro-7-methanesulfonyl-2-morpholin-4-yl-6,7-dihydro-5H-pyrrolo[2,3-d]pyrimidine

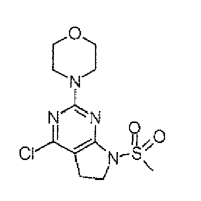

Methane sulfonamide (4.76 g, 50 mmol) and potassium carbonate (3.45 g, 25 mmol) were suspended in NMP (10 ml), and, under stirring at 70°C, NMP solution (20 ml) of 4-[4,6-dichloro-5-(2-chloroethyl)-pyrimidin-2-yl]-morpholine prepared according to Example 1, Step B was added dropwise over 5 minutes. The reaction, mixture was stirred at 100°C for 5 hours, followed by allowing to cool to 25°C, and further stirred for 12 hours. To the above reaction mixture warmed to 70°C, water (30 ml) was added dropwise under stirring while maintaining the internal temperature of the reaction mixture at 70°C. After confirmation by visual inspection that no insolubles were observed in the reaction mixture, it was allowed to cool to 25°C (crystallization was initiated at 50°C). To the obtained suspension, water (30 ml) was further added under stirring, followed by stirring for 30 minutes. The resulting precipitate was filtered, and washed with water (10 ml), to obtain a colorless powder of 4-chloro-7-methanesulfonyl-2-morpholin-4-yl-6,7-dihydro-5H-pyrrolo[2,3-d]pyrimidine (2.13 g, 67%).
¹H-NMR (CDCl₃) δ: 4.08 (2H, dd, J = 9.0, 7.8 Hz), 3.80-3.71 (8H, m), 3.29 (3H, s), 3.02 (2H, dd, J = 9.0, 7.8 Hz).
ESI (LC-MS positive mode) m/z 319, 321 (M+H)⁺.

### Step B

### [5-(7-Methanesulfonyl-2-morpholin-4-yl-6,7-dihydro-5H-pyrrolo[2,3-d]pyrimidin-4-yl)-pyrimidin-2-yl]-bis-(4-methoxy-benzyl)-amine

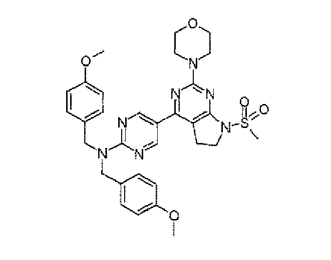

4-Chloro-7-methanesulfonyl-2-morpholin-4-yl-6,7-dlhydro-5H-pyrrolo[2,3-d]pyrimidine (80.0 mg, 0.251 mmol) prepared in Step A, bis-(4-methoxy-benzyl)-[5-(4,4,5,5-tetramethyl-[1,3,2]dioxaborolan-2-yl)-pyrimidin-2-yl]-amine (138.9 mg, 0.301 mmol), potassium phosphate (213 mg, 1.00 mmol) and dichlorobis(triphenylphosphine)palladium (II) (1.8 mg, 2.56 µmol) were diossolved in water/dimethylformamide mixed solvent (1 : 15, 800 µl), and this was degassed under ultrasonic irradiation, followed by stirring at 60°C for 2 hours under an argon atmosphere. After the reaction mixture was cooled to room temperature, water (800 µl) was added, and stirred at room temperature for 30 minutes. The precipitated solid was filtered, and washed sequentially with water (1.5 ml), methanol (1.5 ml), t-butylmethylether (1.5 ml), to obtain [5-(7-methanesulfonyl-2-morpholin-4-yl-6,7-dihydro-5H-pyrrolo[2,3-d]pyrimidin-4-yl)-pyrimidin-2-yl]-bis-(4-methoxy-benzyl)-amine as a white solid (151.6 mg, 98%).
¹H-NMR (DMSO-D₆) δ: 8.97 (2.0H, s), 7.20 (4.0H, d, J = 8.6 Hz), 6.88 (4.0H, d, J = 8.6 Hz), 4.79 (4.0H, s), 3.99 (2.0H, t, J = 8.2 Hz), 3.73 (6.0H, s), 3.75-3.68 (8.0H, m), 3.34 (3.0H, s), 3.27 (1.9H, t, J = 8.2 Hz).
ESI (LC-MS positive mode) m/z 618 (M+H)⁺.
Further, in the present step, using 2-amino-5-(4,4,5,5-tetramethyl-1,3,2-dioxaborolan-2-yl)pyrimidine instead of bis-(4-methoxy-benzyl)-[5-(4,4,5,5-tetramethyl-[1,3,2]dioxaborolan-2-yl)-pyrimidin-2-yl]-amine, 5-(7-methanesulfonyl-2-morpholin-4-yl-6,7-dihydro-5H-pyrrolo[2,3-d]pyrimidin-4-yl)-pyrimidin-2-ylamine was synthesized. 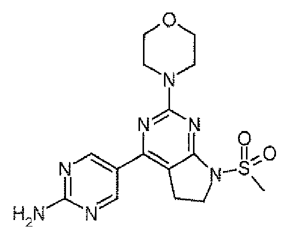 To a DMF solution (550 ml) of 4-chloro-7-methanesulfonyl-2-morpholin-4-yl-6,7-dihydro-5H-pyrrolo[2,3-d]pyrimidine (57.9 g, 181.6 mmol) prepared according to Example 4, Step A, 2-amino-5-(4,4,5,5-tetramethyl-1,3,2-dioxaborolan-2-yl)pyrimidine (48.2 g, 218.0 mmol), potassium phosphate (42.4 g, 199.8 mmol) and water (30 ml) were added, and degassed under a nitrogen atpmosphere. Further, dichlorobis(triphenylphosphine)palladium (II) (1.3 g, 1.8 mmol) was added, and degassed under a nitrogen atomosphere, followed by stirring at 60°C for 2 hours. After the reaction mixture was cooled under ice cooling, water (750 ml) was added, and stirred for 2 hours. The resulting precipitate was filtered, and washed with water (240 ml), acetone (240 ml), to obtain a crude solid (75.6 g). The obtained solid (62.0 g) was suspended in water (1500 ml), and stirred at 50°C for 1 hour. The suspension was filtered, and washed with water (400 ml), acetone (400 ml), to obtain 5-(7-methanesulfonyl-2-morpholin-4-yl-6,7-dihydro-5H-pyrrolo[2,3-d]pyrimidin-4-yl)-pyrimidin-2-ylamine as a grey-white solid (53.7 g, 96%).
ESI (LC-MS positive mode) m/z 378[(M+H)⁺].
Using 5-(7-methanesulfonyl-2-morpholin-4-yl-6,7-dihydro-5H-pyrrolo[2,3-d]pyrimidin-4-yl)-pyrimidin-2-ylamine instead of [5-(7-methanesulfonyl-2-morpholin-4-yl-6,7-dihydro-5H-pyrrolo[2,3-d]pyrimidin-4-yl)-pyrimidin-2-yl]-bis-(4-methoxy-benzyl)-amine in Step C described below, an oxidation reaction according to Step C may be carried out, to obtain 5-(7-methanesulfonyl-2-morpholin-4-yl-7H-pyrrolo[2,3-d]pyrimidin-4-yl)-pyrimidin-2-ylamine.

### Step C

### [5-(7-Methanesulfonyl-2-morpholin-4-yl-7H-pyrrolo[2,3-d]pyrimidin-4-yl)-pyrimidin-2-yl]-bis-(4-methoxy-benzyl)-amine

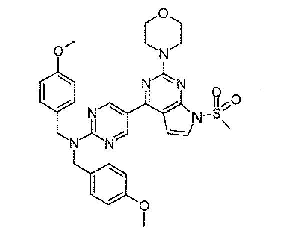

To a dichloromethane solution (3 ml) of [5-(7-methanesulfonyl-2-morpholin-4-yl-6,7-dihydro-5H-pyrrolo[2,3-d]pyrimidin-4-yl)-pyrimidin-2-yl]-bis-(4-methoxy-benzyl)-amine (101.3 mg, 0.164 mmol) prepared in Step B, DDQ (48.4 mg, 0.213 mmol) was added, and stirred at room temperature for 30 minutes. Saturated aqueous sodium chloride solution (25 ml) was added to the reaction mixture, followed by extraction with dichloromethane (50 ml), and the organic layer was dried over magnesium sulfate. After the drying agent was filtered off, the filtrate was concentrated under reduced pressure, and the resulting residue was purified by silica gel column chromatography (dichloromethane/methanol=150/1), to obtain [5-(7-methanesulfonyl-2-morpholin-4-yl-7H-pyrrolo[2,3-d]pyrimidin-4-yl)-pyrimidin-2-yl]-bis-(4-methoxy-benzyl)-amine as a yellow solid (84.5 mg, 84%).
¹H-NMR (CDCl₃) δ: 9.10 (2H, s), 7.33 (1H, d, J = 4.1 Hz), 7.21 (4H, d, J = 8.3 Hz), 6.87 (4H, d, J = 8.3 Hz), 6.72 (1H, d, J = 4.1 Hz), 4.86 (4H, br s), 3.95-3.88 (4H, m), 3.85-3.78 (10H, m), 3.56 (3H, s).
ESI (LC-MS positive mode) m/z 616 (M+H)⁺.

### Step D

### 5-(7-Methanesulfonyl-2-morpholin-4-yl-7H-pyrrolo[2,3-d]pyrimidin-4-yl)-pyrimidin-2-ylamine

To an ethyl aetate solution (3 ml) of [5-(7-methanesulfonyl-2-morpholin-4-yl-7H-pyrrolo[2,3-d]pyrimidin-4-yl)-pyrimidin-2-yl]-bis-(4-methoxy-benzyl)-amine (81.5 mg, 0.132 mmol) prepared in Step C, concentrated sulfuric acid (0.75 ml, 14.0 mmol) was addaed, and stirred at 70°C for 3 hours. After cooling to room temperature, 10% aqueous potassium phosphate solution (50 ml) was added to the reaction mixture. The precipitated solid was filtered, and washed with water (5 ml). This was suspended in dichloromethane (1 ml), and the resulting solid was filtered, followed by washing with dichloromethane (3 ml). The obtained solid was purified by preparative thin layer chromatography (dichloromethane/methanol=10/1), to obtain 5-(7-methanesulfonyl-2-morpholin-4-yl-7H-pyrrolo[2,3-d]pyrimidin-4-yl)-pyrimidin-2-ylamine as a pale yellow solid (22.5 mg, 45%).
¹H-NMR (DMSO-D₆) δ: 9.00 (2H, s), 7.39 (1H, dd, J = 4.1, 1.2 Hz), 7.32 (2H, br s), 7.01 (1H, dd, J = 4.1, 1.2 Hz), 3.84-3.70 (11H, m).
ESI (LC-MS positive mode) m/z 376 (M+H)⁺.

### [Test Example 1]

### [Measurement of PI3K Inhibitory Activity]

The inhibitory activity of compounds of the present invention represented by formula (I) was measured with human PI3K (p110α/p85α) prepared in a baculovirus expression system using the AlphaScreen GST Detection Kit (Perkin Elmer, Inc.). A predetermined concentration of the compound of the present invention dissolved in dimethylsulfoxide (DMSO) and PI3K were mixed in a 384-well assay plate and after allowing to stand for 20 minutes at room temperature, 4 µM PI(4,5)P2 (Echelon Corporation) and 10 µM ATP (5 mM Hepes, pH 7.5, 2.5 mM MgCl₂) were added to initiate the reaction. After reacting at 37°C for 15 minutes, GST-GRP1 expressed and purified from Escherichia coli, Anti-GST Acceptor Beads (Perkin Elmer, Inc.), Streptavidin Donor Beads (Perkin Elmer, Inc.) and biotin-PI (3,4,5)P3 (Echelon Corporation) (10 mM Tris-HCl pH 7.4, 150 mM NaCl, 7.5 mM EDTA, 1 mM DTT, 0.1 % Tween 20) were added, and after allowing to stand for 1 hour at room temperature, light at 520 to 620 nm emitted as a result of exciting with light at 680 nm was measured with the EnVision measuring instrument (Perkin Elmer, Inc.).
The inhibitory activity of the compounds was calculated by assigning a value of 0% inhibitory activity to the measured value following addition of DMSO alone, assigning a value of 100% inhibitory activity to the measured value in the absence of ATP, and defining the concentration that resulted in 50% inhibitory activity as the IC₅₀ value (µM).
Test Example 1 described above can be performed according to "Analytical Biochemistry, 2003, 313, 234-245; Alexander Gray et al".

### [Test Example 2]

### [Measurement of Cell Proliferation Inhibitory Activity]

The cell proliferation inhibitory activity was measured for compounds of the present invention represented by formula (I). Cancer cell proliferation inhibitory activity was measured using the Cell Counting Kit-8 (Dojindo). 2000 cells each of human colon cancer cell line HCT116 purchased from the American Type Culture Collection (Virginia, USA) were seeded into each well of a 96-well culture plate followed by the addition of a predetermined concentration of the compounds and cultivating in a 5% CO₂ environment at 37°C for 4 days. On the fourth day of cultivating, the Cell Counting Kit-8 solution was added and absorbance (measuring wavelength: 450 nm, reference wavelength: 615 nm) was measured in accordance with the protocol provided with the kit. The calculation was carried out by assigning a value of 0% inhibition to the measured value in the case of not containing a test compound, assigning a value of 100% inhibition to the measured value in the case of not containing a test compound and cells, and defining the concentration that resulted in 50% inhibitory activity as the IC₅₀ value (µM).
Cancer cell proliferation inhibitory activity was also measured for human lung cancer cell line NCI-H460 and human prostate cancer cell line PC3 purchased from the American Type Culture Collection. 1000 and 3000 cells of NCI-H460 and PC3, respectively, were seeded into each well of a 96-well culture plate followed by testing in the same manner as the human colon cancer cell line, and the calculation was carried out by defining the concentration that resulted in 50% inhibitory activity as the IC₅₀ value (µM).
The enzyme inhibitory activities and cell proliferation inhibitory activities are shown in the following table. As shown in Table, the compounds of the present invention demonstrated satisfactory enzyme inhibitory activity and cell proliferation inhibitory activity.

| Compound No. | Enzyme inhibitory activity | Cell proliferation Inhibitory activity | | |
|---|---|---|---|---|
| | (IC₅₀, µ M) | (IC₅₀, µ M) | | |
| | PI3K α | Colon cancer (HCT116) | Prostate cancer (PC3) | Non-small cell cancer (NCIH460) |
| 1-1 | 0.007 | 0.12 | 0.18 | 0. 071 |
| 2-1 | 0.093 | 1.45 | 3.18 | 1.25 |
| 3-1 | 0.093 | 0.57 | 0.43 | 0.17 |
| 4-1 | 0.016 | 0.41 | 1.14 | 0.29 |

## Claims

1. A compound represented by the following formula (I): 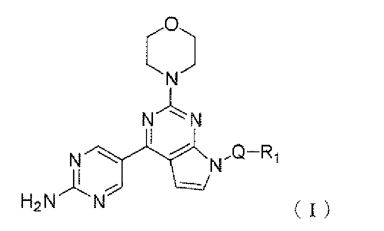 [wherein,
Q represents a linking group represented by -X-Y-;
X represents a single bond, -CO-, -CONH-,-CON(C₁₋₄alkyl)-, -CS-, - CSNH-, -CSN(C₁₋₄ alkyl)- or -SO₂-;
Y represents a single bond, arylene or heteroarylene (the arylene and heteroarylene may be unsubstituted or substituted at 1 to 4 locations by -halogen, -C₁₋₆ alkyl, -OH, or -OC₁₋₆ alkyl);
provided that X and Y are not simultaneously single bonds;
R₁ represents -C₀₋₆ alkylene-(A)ₘ-C₁₋₆ alkyl, or -C₀₋₆ alkylene-(A)ₘ-C₀₋₆ alkylene-(heterocycle);
A represents -CO-, -CS-, -CONH-, -CON(C₁₋₄ alkyl)-, -CSNH-, -CSN(C₁₋₄ alkyl)-, -NH-, or -N(C₁₋₄ alkyl)-;
m represents 0 or 1;
the aforementioned -(heterocycle) is heteroaryl, or a group represented by the following formula (a); 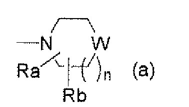 wherein Rₐ and R_{b} are the same or different and represent a hydrogen atom, -C₁₋₆ alkyl, -halogen, -OH, or -OC₁₋₆ alkyl;
W represents -CR_{c}R_{d}-, -O-, -S-, -SO-, -SO₂-, or -NRₑ-; n represents 0 or 1;
R_{c} and R_{d} are the same or different and represent a hydrogen atom, - halogen, -C₁₋₆ alkyl, -OH, -OC₁₋₆ alkyl, or heteroaryl;
Rₑ represents a hydrogen atom, -C₁₋₆ alkyl, -OH, -OC₁₋₆ alkyl, or heteroaryl (-C₁₋₆ alkyl and -OC₁₋₆ alkyl in R_{c}, R_{d} and Rₑ may be substituted by -halogen, or -OH)] or a pharmaceutically acceptable salt thereof.

2. The compound according to claim 1, wherein either X or Y in formula (I) is a single bond,
or a pharmaceutically acceptable salt thereof.

3. The compound according to claim 1, wherein X is a single bond, -CO-, -CONH-,-CSNH-, or -SO₂-,
or a pharmaceutically acceptable salt thereof.

4. The compound according to claim 1, wherein arylene or heteroarylene represented by Y in formula (I) is derived from a ring selected from benzene, pyrrole, pyrazole, imidazole, triazole, oxazole, isoxazole, indazole, thiazole, pyridine, piridazine, pyrimidine, pyrazine, oxazine, triazine, indole, benzimidazole, benzoxazole, benzothiazole, benzopyrazole, quinoline, isoquinoline, quinoxaline, quinazoline, phthalazine, purine, and pteridine,
or a pharmaceutically acceptable salt thereof

5. The compound according to claim 1, wherein -(heterocycle) in R₁ in formula (I) is pyridyl or a group represented by the following formula (a-1), (a-2), (a-3), (a-4), (a-5), (a-6), (a-7), or (a-8): 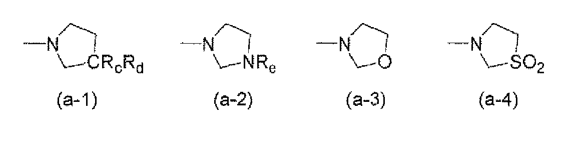 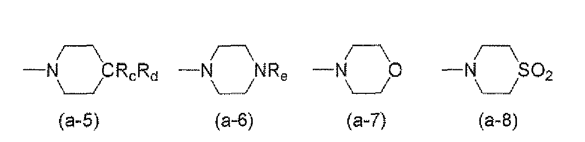 [wherein, R_{c}, R_{d}, and Rₑ are the same as defined in claim 1] or a pharmaceutically acceptable salt thereof.

6. The compound according to claim 1,
wherein X in formula (I) is a single bond, -CO-, -CONH-,-CSNH-, or -SO₂- ; Y is a single bond, phenylene, or pyridinylene; and
R₁ is 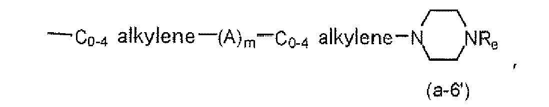 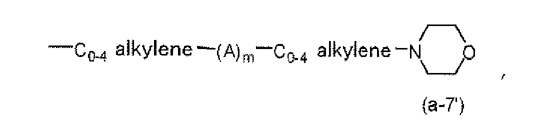 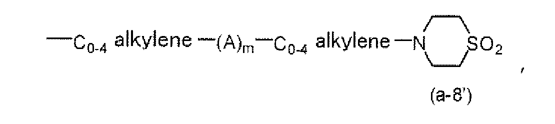 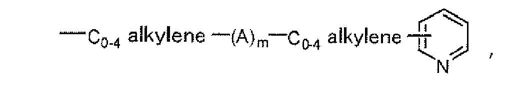 or -C₁₋₄ alkyl
[wherein A represents -CO-, -NH-, -CONH-, or -CONMe- ;
m is 0 or 1 ;
Rₑ is a hydrogen atom, -(C₁₋₆ alkyl which may be substituted by a halogen atom), or pyridyl];
or a pharmaceutically acceptable salt thereof.

7. The compound according to claim 1, selected from
5-{7-[2-(4-ethyl-piperazin-1-yl)-pyridin-4-yl]-2-morpholin-4-yl-7H-pyrrolo[2,3-d]pyrimidin-4-yl}-pyrimidin-2-ylamine;
{3-[4-(2-amino-pyrimidin-5-yl)-2-morpholin-4-yl-pyrrolo[2,3-d]pyrimidin-7-yl]-4-methyl-phenyl}-morpholin-4-yl-methanone;
5-{7-[4-(1,1-dioxo-1λ⁶-thiomorpholin-4-ylmethyl)-phenyl]-2-morpholin-4-yl-7H-pyrrolo[2,3-d]pyrimidin-4-yl} -pyrimidin-2-ylamine; and
5-(7-methanesulfonyl-2-morpholin-4-yl-7H-pyrrolo[2,3-d]pyrimidin-4-yl)-pyrimidin-2-ylamine,
or a pharmaceutically acceptable salt thereof.

8. A process for preparing a compound represented by formula (I) according to claim 1: 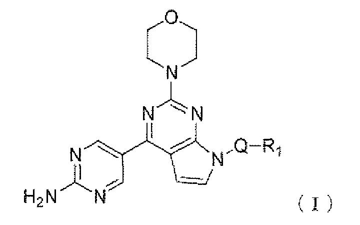 [wherein, Q and R₁ are the same as defined in claim 1]
which comprises the step of reacting the compound represented by formula (VIa): 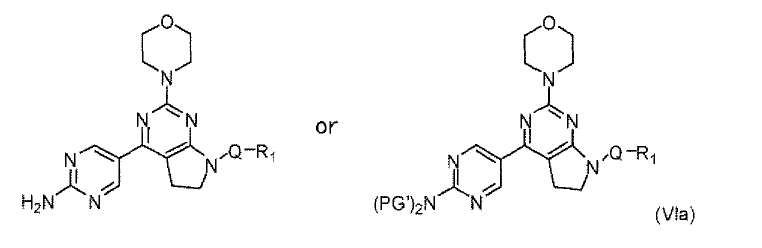 [wherein, Q and R₁ are the same as defined in claim 1; and PG' represents an amino group-protecting group]
with an oxidizing agent, and may further comprise the step of removing the amino group-protecting group.

9. A pharmaceutical composition comprising as an active ingredient the compound according to any of claims 1 to 7 or a pharmaceutically acceptable salt thereof.

10. A PI3K inhibitor comprising as an active ingredient the compound according to any of claims 1 to 7 or a pharmaceutically acceptable salt thereof.

11. A preventive agent or therapeutic agent of a proliferative disease comprising as an active ingredient the compound according to any of claims 1 to 7 or a pharmaceutically acceptable salt thereof.

12. The preventive agent or therapeutic agent according to claim 11, wherein the proliferative disease is cancer.

13. The preventive agent or therapeutic agent according to claim 12, wherein the cancer is colon cancer, prostate cancer or non small cell lung cancer.
